# EUROPEAN PATENT SPECIFICATION

(11) **EP 1 644 367 B1**
(45) Date of publication and mention of the grant of the patent: **14.10.2015**
(21) Application number: 04752676.9
(22) Date of filing: 19.05.2004
(51) Int. Cl.: C07D 217/16, C07D 413/04, C07D 217/24

(54) **IMMUNOSUPPRESSANT COMPOUNDS AND COMPOSITIONS**
DIE IMMUNREAKTION UNTERDRÜCKENDE VERBINDUNGEN UND ZUSAMMENSETZUNGEN
COMPOSITIONS ET COMPOSES IMMUNOSUPPRESSEURS

(30) Priority: 19.05.2003 US 471931 P; 14.04.2004 US 562182 P
(43) Date of publication of application: 12.04.2006
(73) Proprietor: Novartis AG, 4056 Basel (CH)
(72) Inventor: PAN, Shifeng, San Diego, CA 92130 (US); GRAY, Nathanael S., San Diego, CA 92122 (US); FAN, Yi, Poway, CA 92064 (US); GAO, Wenqi, San Diego, CA 92123 (US); MI, Yuan, San Diego, CA 92131 (US)
(74) Representative: Wiessner, Michael
(86) International application number: PCT/US2004/015699
(87) International publication number: WO 2004/113330

(56) References cited:
- WO-A-03/091219
- WO-A-2004/014856
- DATABASE CA [Online] CHEMICAL ABSTRACTS SERVICE, COLUMBUS, OHIO, US; FISHER, MATTHEW J. ET AL: "Dihydroisoquinolone RGD mimics. Exploration of the aspartate isostere" XP002504798 retrieved from STN Database accession no. 1997:723317 & BIOORGANIC & MEDICINAL CHEMISTRY LETTERS , 7(19), 2537-2542 CODEN: BMCLE8; ISSN: 0960-894X, 1997,
- SMITH C.D. ET AL: 'Electrospray mass spectrometry of stable iminyl nitroxide and nitronyl nitroxide free radicals' JOURNAL OF MASS SPECTROMETRY vol. 37, no. 9, 2002, pages 897 - 902, XP002982157

## Description

### BACKGROUND OF THE INVENTION

### Field of the Invention

The invention provides a novel class of immunosuppressant compounds useful in the treatment or prevention of diseases or disorders mediated by lymphocyte interactions, particularly diseases associated with EDG receptor mediated signal transduction.

### Background

EDG receptors belong to a family of closely related, lipid activated G-protein coupled receptors. EDG-1, EDG-3, EDG-5, EDG-6, and EDG-8 (also respectively termed S1P1, S1P3, S1P2, S1P4, and S1P5) are identified as receptors specific for sphingosine-1-phosphate (S1P). EDG2, EDG4, and EDG7 (also termed LPA1, LPA2, and LPA3, respectively) are receptors specific for lysophosphatidic (LPA). Among the S1P receptor isotypes, EDG-1, EDG-3 and EDG-5 are widely expressed in various tissues, whereas the expression of EDG-6 is confined largely to lymphoid tissues and platelets, and that of EDG-8 to the central nervous system. EDG receptors are responsible for signal transduction and are thought to play an important role in cell processes involving cell development, proliferation, maintenance, migration, differentiation, plasticity and apoptosis. Certain EDG receptors are associated with diseases mediated by lymphocyte interactions, for example, in transplantation rejection, autoimmune diseases, inflammatory diseases, infectious diseases and cancer. An alteration in EDG receptor activity contributes to the pathology and/or symptomology of these diseases. Accordingly, molecules that themselves alter the activity of EDG receptors are useful as therapeutic agents in the treatment of such diseases. Document EP 1430894 A1 discloses 7-(benzenesulfonamide)indole derivatives as lymphocytic activation inhibitors for use in the treatment of autoimmnune and inflammatory diseases.

### SUMMARY OF THE INVENTION

This application relates to compounds of Formula I: 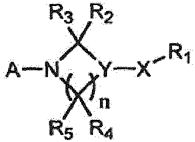 in which:
- n: is 1 or 2;
- A: is -(CH₂)₂C(O)OH;
- X: is a bond or is selected from [1,2,4]oxadiazole, -CH₂O-, -OCH₂-, isoxazoles and [1,3,4]oxadiazole;
- Y: is selected from phenyl and benzooxazolyl;
- R₁: is chosen from C₆₋₁₀aryl and C₂₋₉heteroaryl; wherein any aryl or heteroaryl of R₁ is optionally substituted by a radical chosen from C₆₋₁₀arylC₀₋₄alkyl, C₂₋₉heteroarylC₀₋₄alkyl, C₃₋₈cycloalkylC₀₋₄alkyl, C₃₋₈heterocycloalkylC₀₋₄alkyl or C₁₋₆alkyl; wherein any aryl, heteroaryl, cycloalkyl or heterocycloalkyl group of R₁ can be optionally substituted by one to five radicals chosen from halo, C₁₋₆alkyl, C₁₋₆alkoxy, halo-substituted-C₁₋₆alkyl and halo-substituted-C₁₋₆alkoxy; and any alkyl group of R₁ can optionally have a methylene replaced by an atom or group chosen from -S-, -S(O)-, -S(O)₂-, -NR₇- and -O-; wherein R₇ is chosen from hydrogen or C₁₋₆alkyl;
- R₂, R₃, R₄ and R₅: are hydrogen; and the pharmaceutically acceptable salts, hydrates, solvates, N-oxide derivatives, optical or geometric isomers selected from cis-compounds, trans-compounds and mixtures thereof.

A second aspect of the invention is a pharmaceutical composition which contains a compound of Formula I or an N-oxide derivative, individual optical isomer or a pharmaceutically acceptable salt thereof, in admixture with one or more suitable excipients.

The invention discloses compounds of Formula (I) for use in a method for treating a disease in an animal in which alteration of EDG receptor mediated signal transduction can prevent, inhibit or ameliorate the pathology and/or symptomology of the disease, which method comprises administering to the animal a therapeutically effective amount of a compound of Formula I or a N-oxide derivative, individual isomer or mixture of isomers thereof; or a pharmaceutically acceptable salt thereof.

A fourth aspect of the invention is the use of a compound of Formula I in the manufacture of a medicament for treating a disease in an animal in which alteration of EDG receptor mediated signal transduction contributes to the pathology and/or symptomology of the disease. geometric isomers selected from cis-compounds, trans compounds and mixtures thereof.

### DESCRIPTION OF THE PREFERRED EMBODIMENTS

The invention provides compounds that are useful in the treatment and/or prevention of diseases or disorders mediated by lymphocyte interactions. Also provided are methods for treating such diseases or disorders.

### Definitions

In this specification, unless otherwise defined:
"Alkyl" as a group and as a structural element of other groups, for example halo-substituted-alkyl, alkoxy, acyl, alkylthio, alkylsulfonyl and alkylsulfinyl, can be either straight-chained or branched. "Alkenyl" as a group and as a structural element of other groups contains one or more carbon-carbon double bonds, and can be either straight-chain, or branched. Any double bonds can be in the cis- or trans- configuration. "Alkynyl" as a group and as structural element of other groups and compounds contains at least one C ≡C triple bond and can also contain one or more C=C double bonds, and can, so far as possible, be either straight-chain or branched. Any cycloalkyl group, alone or as a structural element of other groups can contain from 3 to 8 carbon atoms, preferably from 3 to 6 carbon atoms. "Alkylene" and "alkenylene" are divalent radicals derived from "alkyl" and "alkenyl" groups, respectively. In this application, any alkyl group of R¹ can be optionally interrupted by a member of the group selected from -S-, -S(O)-, -S(O)₂-, NR²⁰- and -O- (wherein R²⁰ is hydrogen or C₁₋₆alkyl). These groups include -CH₂-O-CH₂-, -CH₂-S(O)₂-CH₂-, - (CH₂)₂-NR²⁰-CH₂-, -CH₂-O-(CH₂)₂-, and the like.
"Aryl" means a monocyclic or fused bicyclic aromatic ring assembly containing six to ten ring carbon atoms. For example, C₆₋₁₂aryl can be phenyl, biphenyl or naphthyl, preferably phenyl. A fused bicyclic ring can be partially saturated, for example, 1,2,3,4-tetrahydro-naphthalene, and the like. "Arylene" means a divalent radical derived from an aryl group. For example, arylene as used in this application can be phenylene, biphenylene, naphthylene and the like.
"Halo" or "halogen" means F, Cl, Br or I, preferably F or Cl. Halo-substituted alkyl groups and compounds can be partially halogenated or perhalogenated, whereby in the case of multiple halogenation, the halogen substituents can be identical or different. A preferred perhalogenated alkyl group is for example trifluoromethyl or trifluoromethoxy.
"Heteroaryl" means aryl, as defined in this application, with the addition of at least one heteroatom moiety selected from N, O or S, and each ring is comprised of 5 to 6 ring atoms, unless otherwise stated. For example, C₂heteroaryl includes oxadiazole, triazole, and the like. C₉heteroaryl includes quinoline, 1,2,3,4-tetrahydro-quinoline, and the like. C₂₋₉heteroaryl as used in this application includes thienyl, pyridinyl, furanyl, isoxazolyl, benzoxazolyl or benzo[1,3]dioxolyl, preferably thienyl, furanyl or pyridinyl. "Heteroarylene" means heteroaryl, as defined in this application, provided that the ring assembly comprises a divalent radical. A fused bicyclic heteroaryl ring system can be partially saturated, for example, 2,3-dihydro-1H-isoindole, 1,2,3,4-tetrahydro-quinoline, and the like.

As used in the present invention, an EDG-1 selective compound (agent or modulator) has a specificity that is selective for EDG-1 over EDG-3 and over one or more of EDG-5, EDG-6, and EDG-8. As used herein, selectivity for one EDG receptor (a "selective receptor") over another EDG receptor (a "non-selective receptor") means that the compound has a much higher potency in inducing activities mediated by the selective EDG receptor (e.g., EDG-1) than that for the non-selective S1P-specific EDG receptor. If measured in a GTP-γS binding assay (as described in the Example below), an EDG-1 selective compound typically has an EC50 (effective concentration that causes 50% of the maximum response) for a selective receptor (EDG-1) that is at least 5, 10, 25, 50,100, 500, or 1000 fold lower than its EC50 for a non-selective receptor (e.g., one or more of EDG-3, EDG-5, EDG-6, and EDG-8).

### Detailed Description of the Invention

The invention provides compounds that are useful for treating or preventing diseases or disorders that are mediated by lymphocyte interactions. In one embodiment, for compounds of Formula I, R₁ is phenyl, naphthyl, furanyl or thienyl optionally substituted by C₆₋₁₀arylC₀₋₄alkyl, C₂₋₉heteroarylC₀₋₉alkyl, C₃₋₈cycloalkylC₀₋₄alkyl, C₃₋₈heterocycloalkylC₀₋₄alkyl or C₁₋₆alkyl; wherein any aryl, heteroaryl, cycloalkyl or heterocycloalkyl group of R₁ can be optionally substituted by one to five radicals chosen from halo, C₁₋₆alkyl, C₁₋₆alkoxy, halo-substituted-C₁₋₆alkyl and halo-substituted-C₁₋₆alkoxy; and any alkyl group of R₁ can optionally have a methylene replaced by an atom or group chosen from -S-, -S(O)-,-S(O)₂-, -NR₇- and -O-; wherein R₇ is hydrogen or C₁₋₆alkyl.

In another embodiment, R₁ is chosen from: 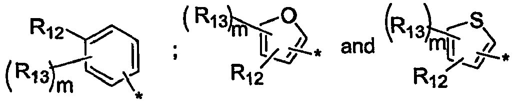 wherein the asterisk is the point of attachment of R₁ with X; m is chosen from 1 and 2; R₁₂ is selected from hydrogen, C₆₋₁₀arylC₀₋₄alkyl, C₂₋₉heteroarylC₀₋₄alkyl, C₃₋₈cycloalkylC₀₋₄alkyl, C₃₋₈heterocycloalkylC₀₋₄alkyl or C₁₋₆alkyl; wherein any aryl, heteroaryl, cycloalkyl or heterocycloalkyl group of R₁₂ can be optionally substituted by one to three radicals chosen from halo, C₁₋₆alkyl, C₁₋₆alkoxy, halo-substituted-C₁₋₆alkyl and halo-substituted-C₁₋₆alkoxy; and any alkyl group of R₁₂ can optionally have a methylene replaced by an atom or group chosen from -S-, -S(O)-, -S(O)₂-, -NR₁₀- and -O-; wherein R₁₀ is hydrogen or C₁₋₆alkyl; and R₁₃ is chosen from halo, C₁₋₆alkyl, C₁₋₆alkoxy, halo-substituted-C₁₋₆alkyl and halo-substituted-C₁₋₆alkoxy.

In another embodiment, R₁ is selected from: 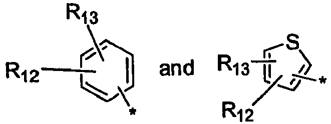 wherein R₁₂ is selected from hydrogen, phenyl and cyclohexyl; wherein any phenyl or cyclohexyl of R₁₂ is optionally substituted with methyl; and R₁₃ is selected from trifluoromethyl, methyl and ethyl.

Preferred compounds of the invention are selected from 3-{6-[3-(2-trifluoromethyl-biphenyl-4-yl)-[1,2,4]oxadiazol-5-yl]-3,4-dihydro-1H-isoquinolin-2-yl}-propionic acid, 3-[6-(2-trifluoromethyl-biphenyl-4-yloxymethyl)-3,4-dihydro-1H-isoquinolin-2-yl]-propionic acid, 3-{5-[5-(2-trifluoromethyl-biphenyl-4-yl)-isoxazol-3-yl]-1,3-dihydro-isoindol-2-yl}-propionic acid, 3-{5-[5-(2-trifluoromethyl-biphenyl-4-yl)-[1,2,4]oxadiazol-3-yl]-1,3-dihydro-isoindol-2-yl}-propionic acid, 3-{5-[5-(2-trifluoromethyl-biphenyl-4-yl)-[1,3,4]oxadiazol-2-yl]-1,3-dihydro-isoindol-2-yl}-propionic acid, 3-[2-(2-trifluoromethyl-biphenyl-4-yl)-5,7-dihydro-oxazolo[4,5-f]isoindol-6-yl]-propionic acid, 3-{7-[5-(2-trifluoromethyl-biphenyl-4-yl)-[1,2,4]oxadiazol-3-yl]-3,4-dihydro-1H-isoquinolin-2-yl}-propionic acid, 3-{6-[5-(2-trifluoromethyl-biphenyl-4-yl)-[1,2,4]oxadiazol-3-yl]-3,4-dihydro-1H-isoquinolin-2-yl}-propionic acid, 3-{6-[5-(2-trifluoromethyl-biphenyl-4-yl)-[1,3,4]oxadiazol-2-yl]-3,4-dihydro-1H-isoquinolin-2-yl}-propionic acid, 3-[6-(3-trifluoromethyl-benzyloxy)-3,4-dihydro-1H-isoquinolin-2-yl]-propionic acid, 3-[6-(4-cyclohexyl-3-trifluoromethyl-henzyloxy)-3,4-dihydro-1H-isoquinolin-2-yl]-propionic acid, 3-[6-(2-trifluoromethyl-biphenyl-4-ylmethoxy)-3,4-dihydry-1H-isoquinolin-2-yl]-prapionic acid, 3-[7-(4-cyclohexyl-3-trifluoromethyl-benzyloxy)-3,4-dihydro-1H-isoquinolin-2-yl]-propionic acid, 3-[6-(4-cyclohexyl-3-methyl-phenoxymethyl)-3,4-dihydro-1H-isoquinolin-2-yl]-propionic acid, 3-[6-(4-cyclohexyl-3-ethyl-phenoxymethyl)-3,4-dihydro-1H-isoquinolin-2-yl]-propionic acid, 3-[6-(2-ethyl-biphenyl-4-yloxymethyi)-3,4-dihydro-1H-isoquinolin-2-yl]-propionic acid and 3-[6-(2-ethyl-3'-methyl-biphenyl-4-yloxymethyl)-3,4-dihydro-1H-isoquinolin-2-yl]-propionic acid.

Further preferred compounds are also shown in the examples and table 1, infra. Forms of the compound that have the hydroxyl or amine group present in a protected form; these function as prodrugs. Prodrugs are compounds that are converted into an active drug form after administration, through one or more chemical or biochemical transformations. Forms of the compounds of the present invention that are readily converted into the claimed compound under physiological conditions are prodrugs of the claimed compounds. Examples of prodrugs include forms where a hydroxyl group is acylated to form a relatively labile ester such as an acetate ester, and forms where an amine group is acylated with the carboxylate group of glycine or an L-amino acid such as serine, forming an amide bond that is particularly susceptible to hydrolysis by common metabolic enzymes.

Compounds of Formula I can exist in free form or in salt form, e.g. addition salts with inorganic or organic acids. Where hydroxyl groups are present, these groups can also be present in salt form, e.g. an ammonium salt or salts with metals such as lithium, sodium, potassium, calcium, zinc or magnesium, or a mixture thereof. Compounds of Formula I and their salts in hydrate or solvate form are also part of the invention.

When the compounds of Formula I have asymmetric centers in the molecule, various optical isomers are obtained. The present invention also encompasses enantiomers, racemates, diastereoisomers and mixtures thereof. Moreover, when the compounds of Formula I include geometric isomers, the present invention embraces cis-compounds, trans-compounds and mixtures thereof. Similar considerations apply in relation to starting materials exhibiting asymmetric carbon atoms or unsaturated bonds as mentioned above.

### Methods and Pharmaceutical Compositions for Treating Immunomodulatory Conditions

The compounds of Formula I in free form or in pharmaceutically acceptable salt form, exhibit valuable pharmacological properties, e.g. lymphocyte recirculation modulating properties, for example, as indicated by the *in vitro* and *in vivo* tests of Example 3 and are therefore indicated for therapy. Compounds of Formula I preferably show an EC₅₀ in the range of 1 x 10⁻¹¹ to 1 x 10⁻⁵ M, preferably less than 50nM. The compounds exhibit selectivity for one or more EDG/S1P receptors, preferably EDG-1/S1P-1. EDG-1/S1P-1 selective modulators of the present invention can be identified by assaying a compound's binding to EDG-1/S1P-1 and one or more of the other EDG/S1P receptors (e.g., EDG-3/S1P-3, EDG-5/S1P-2, EDG-6/S1P-4, and EDG-8/S1P-5). An EDG-1/S1P-1 selective modulator usually has an EC50 for the EDG-1/S1P-1 receptor in the range of 1 x 10⁻¹¹ to 1 x 10⁻⁵ M, preferably less than 50 nM, more preferably less than 5 nM. It also has an EC50 for one or more of the other EDG/S1P receptors that is at least 5, 10, 25, 50, 100, 500, or 1000 fold higher than its EC50 for EDG-1/S1P-1. Thus, some of the EDG-1/S1P-1 modulatory compounds will have an EC50 for EDG-1/S1P-1 that is less than 5 nM while their EC50 for one or more of the other EDG/S 1P receptors are at least 100 nM or higher. Other than assaying binding activity to the EDG/S1P receptors, EDG-1/S1P-1 selective agents can also be identified by examining a test agent's ability to modify a cellular process or activity mediated by an EDG/S1P receptor.

The compounds of formula I are, therefore, useful in the treatment and/or prevention of diseases or disorders mediated by lymphocytes interactions, for example in transplantation, such as acute or chronic rejection of cell, tissue or organ allo- or xenografts or delayed graft function, graft versus host disease, autoimmune diseases, e.g. rheumatoid arthritis, systemic lupus erythematosus, hashimoto's thyroidis, multiple sclerosis, myasthenia gravis, diabetes type I or II and the disorders associated therewith, vasculitis, pernicious anemia, Sjoegren syndrome, uveitis, psoriasis, Graves ophthalmopathy, alopecia areata and others, allergic diseases, e.g. allergic asthma, atopic dermatitis, allergic rhinitis/conjunctivitis, allergic contact dermatitis, inflammatory diseases optionally with underlying aberrant reactions, e.g. inflammatory bowel disease, Crohn's disease or ulcerative colitis, intrinsic asthma, inflammatory lung injury, inflammatory liver injury, inflammatory glomerular injury, atherosclerosis, osteoarthritis, irritant contact dermatitis and further eczematous dermatitises, seborrhoeic dermatitis, cutaneous manifestations of immunologically-mediated disorders, inflammatory eye disease, keratoconjunctivitis, myocarditis or hepatitis, ischemia/reperfusion injury, e.g. myocardial infarction, stroke, gut ischemia, renal failure or hemorrhage shock, traumatic shock, T cell lymphomas or T cell leukemias, infectious diseases, e.g. toxic shock (e.g. superantigen induced), septic shock, adult respiratory distress syndrome or viral infections, e.g. AIDS, viral hepatitis, chronic bacterial infection, or senile dementia. Examples of cell, tissue or solid organ transplants include e.g. pancreatic islets, stem cells, bone marrow, corneal tissue, neuronal tissue, heart, lung, combined heart-lung, kidney, liver, bowel, pancreas, trachea or oesophagus. For the above uses the required dosage will of course vary depending on the mode of administration, the particular condition to be treated and the effect desired.

Furthermore, the compounds of formula I are useful in cancer chemotherapy, particularly for cancer chemotherapy of solid tumors, e.g. breast cancer, or as an anti-angiogenic agent.

The required dosage will of course vary depending on the mode of administration, the particular condition to be treated and the effect desired. In general, satisfactory results are indicated to be obtained systemically at daily dosages of from about 0.03 to 2.5 mg/kg per body weight. An indicated daily dosage in the larger mammal, e.g. humans, is in the range from about 0.5 mg to about 100 mg, conveniently administered, for example, in divided doses up to four times a day or in retard form. Suitable unit dosage forms for oral administration comprise from ca. 1 to 50 mg active ingredient.

The compounds of Formula I can be administered by any conventional route, in particular enterally, for example, orally, e.g. in the form of tablets or capsules, or parenterally, for example, in the form of injectable solutions or suspensions, topically, e.g. in the form of lotions, gels, ointments or creams, or in a nasal or a suppository form. Pharmaceutical compositions comprising a compound of Formula I in free form or in pharmaceutically acceptable salt form in association with at least one pharmaceutical acceptable carrier or diluent can be manufactured in conventional manner by mixing with a pharmaceutically acceptable carrier or diluent.

The compounds of Formula I can be administered in free form or in pharmaceutically acceptable salt form, for example, as indicated above. Such salts can be prepared in a conventional manner and exhibit the same order of activity as the free compounds.

In accordance with the foregoing the present invention further provides compounds of Formula I for use in:
1.1 A method for preventing or treating disorders or diseases mediated by lymphocytes, e.g. such as indicated above, in a subject in need of such treatment, which method comprises administering to said subject an effective amount of a compound of formula I or a pharmaceutically acceptable salt thereof;
1.2 A method for preventing or treating acute or chronic transplant rejection or T-cell mediated inflammatory or autoimmune diseases, e.g. as indicated above, in a subject in need of such treatment, which method comprises administering to said subject an effective amount of a compound of formula I or a pharmaceutically acceptable salt thereof;
1.3 A method for inhibiting or controlling deregulated angiogenesis, e.g. sphingosine-1-phosphate (S1P) mediated angiogenesis, in a subject in need thereof, comprising administering to said subject a therapeutically effective amount of a compound of formula I or a pharmaceutically acceptable salt thereof.
1.4 A method for preventing or treating diseases mediated by a neo-angiogenesis process or associated with deregulated angiogenesis in a subject in need thereof, comprising administering to said subject a therapeutically effective amount of a compound of formula I or a pharmaceutically acceptable salt thereof. The invention also provides:
2. A compound of formula I, in free form or in a pharmaceutically acceptable salt form for use as a pharmaceutical, e.g. in any of the methods as indicated under 1.1 to 1.4 above.
3. A pharmaceutical composition, e.g. for use in any of the methods as in 1.1 to 1.4 above comprising a compound of formula I in free form or pharmaceutically acceptable salt form in association with a pharmaceutically acceptable diluent or carrier therefor.
4. A compound of formula I or a pharmaceutically acceptable salt thereof for use in the preparation of a pharmaceutical composition for use in any of the method as in 1.1 to 1.4 above.

The compounds of formula I may be administered as the sole active ingredient or in conjunction with, e.g. as an adjuvant to, other drugs e.g. immunosuppressive or immunomodulating agents or other anti-inflammatory agents, e.g. for the treatment or prevention of allo- or xenograft acute or chronic rejection or inflammatory or autoimmune disorders, or a chemotherapeutic agent, e.g. a malignant cell anti-proliferative agent. For example the compounds of formula I may be used in combination with a calcineurin inhibitor, e.g. cyclosporin A or FK 506; a mTOR inhibitor, e.g. rapamycin, 40-0-(2-hydroxyethyl)-rapamycin, CCI779, ABT578 or AP23573; an ascomycin having immunosuppressive properties, e.g. ABT-281, ASM981, etc.; corticosteroids; cyclophosphamide; azathioprene; methotrexate; leflunomide; mizoribine; mycophenolic acid; mycophenolate mofetil; 15-deoxyspergualine or an immunosuppressive homologue, analogue or derivative thereof; immunosuppressive monoclonal antibodies, e.g. monoclonal antibodies to leukocyte receptors, e.g. MHC, CD2, CD3, CD4, CD7, CD8, CD25, CD28, CD40. CD45, CD58, CD80, CD86 or their ligands; other immunomodulatory compounds, e.g. a recombinant binding molecule having at least a portion of the extracellular domain of CTLA4 or a mutant thereof, e.g. an at least extracellular portion of CTLA4 or a mutant thereof joined to a non-CTLA4 protein sequence, e.g. CTLA4Ig (for ex. designated ATCC 68629) or a mutant thereof, e.g. LEA29Y ; adhesion molecule inhibitors, e.g. LFA-1 antagonists, ICAM-1 or -3 antagonists, VCAM-4 antagonists or VLA-4 antagonists; or a chemotherapeutic agent.

By the term "chemotherapeutic agent" is meant any chemotherapeutic agent and it includes but is not limited to,
i. an aromatase inhibitor,
ii. an anti-estrogen, an anti-androgen (especially in the case of prostate cancer) or a gonadorelin agonist,
iii. a topoisomerase I inhibitor or a topoisomerase II inhibitor,
iv. a microtubule active agent, an alkylating agent, an antineoplastic antimetabolite or a platin compound,
v. a compound targeting/decreasing a protein or lipid kinase activity or a protein or lipid phosphatase activity, a further anti-angiogenic compound or a compound which induces cell differentiation processes,
vi. a bradykinin 1 receptor or an angiotensin II antagonist,
vii. a cyclooxygenase inhibitor, a bisphosphonate, a histone deacetylase inhibitor, a heparanase inhibitor (prevents heparan sulphate degradation), e.g. PI-88, a biological response modifier, preferably a lymphokine or interferons, e.g. interferon □, an ubiquitination inhibitor, or an inhibitor which blocks anti-apoptotic pathways,
viii. an inhibitor of Ras oncogenic isoforms, e.g. H-Ras, K-Ras or N-Ras, or a farnesyl transferase inhibitor, e.g. L-744,832 or DK8G557,
ix. a telomerase inhibitor, e.g. telomestatin,
x. a protease inhibitor, a matrix metalloproteinase inhibitor, a methionine aminopeptidase inhibitor, e.g. bengamide or a derivative thereof, or a proteosome inhibitor, e.g. PS-341, and/or
xi. a mTOR inhibitor.

The term "aromatase inhibitor" as used herein relates to a compound which inhibits the estrogen production, i.e. the conversion of the substrates androstenedione and testosterone to estrone and estradiol, respectively. The term includes, but is not limited to steroids, especially atamestane, exemestane and formestane and, in particular, non-steroids, especially aminoglutethimide, roglethimide, pyridoglutethimide, trilostane, testolactone, ketokonazole, vorozole, fadrozole, anastrozole and letrozole. A combination of the invention comprising a chemotherapeutic agent which is an aromatase inhibitor is particularly useful for the treatment of hormone receptor positive tumors, e.g. breast tumors.

The term "anti-estrogen" as used herein relates to a compound that antagonizes the effect of estrogens at the estrogen receptor level. The term includes, but is not limited to tamoxifen, fulvestrant, raloxifene and raloxifene hydrochloride. A combination of the invention comprising a chemotherapeutic agent which is an anti-estrogen is particularly useful for the treatment of estrogen receptor positive tumors, e.g. breast tumors.

The term "anti-androgen" as used herein relates to any substance which is capable of inhibiting the biological effects of androgenic hormones and includes, but is not limited to, bicalutamide.

The term "gonadorelin agonist" as used herein includes, but is not limited to abarelix, goserelin and goserelin acetate.

The term "topoisomerase I inhibitor" as used herein includes, but is not limited to topotecan, irinotecan, 9-nitrocamptothecin and the macromolecular camptothecin conjugate PNU-166148 (compound A1 in WO99/17804).

The term "topoisomerase II inhibitor" as used herein includes, but is not limited to the anthracyclines such as doxorubicin, daunorubicin, epirubicin, idarubicin and nemorubicin, the anthraquinones mitoxantrone and losoxantrone, and the podophillotoxines etoposide and teniposide.

The term "microtubule active agent" relates to microtubule stabilizing and microtubule destabilizing agents including, but not limited to taxanes, e.g. paclitaxel and docetaxel, vinca alkaloids, e.g., vinblastine, especially vinblastine sulfate, vincristine especially vincristine sulfate, and vinorelbine, discodermolides and epothilones and derivatives thereof, e.g. epothilone B or a derivative thereof.

The term "alkylating agent" as used herein includes, but is not limited to busulfan, chlorambucil, cyclophosphamide, ifosfamide, melphalan or nitrosourea (BCNU or Gliadel™).

The term "antineoplastic antimetabolite" includes, but is not limited to 5-fluorouracil, capecitabine, gemcitabine, cytarabine, fludarabine, thioguanine, methotrexate and edatrexate.

The term "platin compound" as used herein includes, but is not limited to carboplatin, cis-platin and oxaliplatin.

The term "compounds targeting/decreasing a protein or lipid kinase activity or further anti-angiogenic compounds" as used herein includes, but is not limited to protein tyrosine kinase and/or serine and/or threonine kinase inhibitors or lipid kinase inhibitors, e.g. compounds targeting, decreasing or inhibiting the activity of the epidermal growth factor family of receptor tyrosine kinases (EGFR, ErbB2, ErbB3, ErbB4 as homo- or heterodimers), the vascular endothelial growth factor family of receptor tyrosine kinases (VEGFR), the platelet-derived growth factor-receptors (PDGFR), the fibroblast growth factor-receptors (FGFR), the insulin-like growth factor receptor 1 (IGF-1R), the Trk receptor tyrosine kinase family, the Axl receptor tyrosine kinase family, the Ret receptor tyrosine kinase, the Kit/SCFR receptor tyrosine kinase, members of the c-Abl family and their gene-fusion products (e.g. BCR-Ab1), members of the protein kinase C (PKC) and Raf family of serine/threonine kinases, members of the MEK, SRC, JAK, FAK, PDK or PI(3) kinase family, or of the PI(3)-kinase-related kinase family, and/or members of the cyclin-dependent kinase family (CDK) and anti-angiogenic compounds having another mechanism for their activity, e.g. unrelated to protein or lipid kinase inhibition.

Compounds which target, decrease or inhibit the activity of VEGFR are especially compounds, proteins or antibodies which inhibit the VEGF receptor tyrosine kinase, inhibit a VEGF receptor or bind to VEGF, and are in particular those compounds, proteins or monoclonal antibodies generically and specifically disclosed in WO 98/35958, e.g. 1-(4-chloroanilino)-4-(4-pyridylmethyl)phthalazine or a pharmaceutically acceptable salt thereof, e.g. the succinate, in WO 00/27820, e.g. a N-aryl(thio) anthranilic acid amide derivative e.g. 2-[(4-pyridyl)methyl]amino-N-[3-methoxy-5-(trifluoromethyl)phenyl]benzamide or 2-[(1-oxido-4-pyridyl)methyl]amino-N-[3-trifluoromethylphenyl]benzamide, or in WO 00/09495, WO 00/59509, WO 98/11223, WO 00/27819 and EP 0 769 947; those as described by M. Prewett et al in Cancer Research 59 (1999) 5209-5218, by F. Yuan et al in Proc. Natl. Acad. Sci. USA, vol. 93, pp. 14765-14770, Dec. 1996, by Z. Zhu et al in Cancer Res. 58, 1998, 3209-3214, and by J. Mordenti et al in Toxicologic Pathology, Vol. 27, no. 1, pp 14-21, 1999; in WO 00/37502 and WO 94/10202; Angiostatin™, described by M. S. O'Reilly et al, Cell 79, 1994, 315-328; Endostatin™, described by M. S. O'Reilly et al, Cell 88, 1997, 277-285; anthranilic acid amides; ZD4190; ZD6474; SU5416; SU6668; or anti-VEGF antibodies or anti-VEGF receptor antibodies,e.g. RhuMab.

By antibody is meant intact monoclonal antibodies, polyclonal antibodies, multispecific antibodies formed from at least 2 intact antibodies, and antibody fragments so long as they exhibit the desired biological activity.

Compounds which target, decrease or inhibit the activity of the epidermal growth factor receptor family are especially compounds, proteins or antibodies which inhibit members of the EGF receptor tyrosine kinase family, e.g. EGF receptor, ErbB2, ErbB3 and ErbB4 or bind to EGF or EGF related ligands, or which have a dual inhibiting effect on the ErbB and VEGF receptor kinase and are in particular those compounds, proteins or monoclonal antibodies generically and specifically disclosed in WO 97/02266, e.g. the compound of ex. 39, or in EP 0 564 409, WO 99/03854, EP 0520722, EP 0 566 226, EP 0 787 722, EP 0 837 063, US 5,747,498, WO 98/10767, WO 97/30034, WO 97/49688, WO 97/38983 and, especially, WO 96/30347 (e.g. compound known as CP 358774), WO 96/33980 (e.g. compound ZD 1839) and WO 95/03283 (e.g. compound ZM105180) or PCT/EP02/08780; e.g. trastuzumab (Herpetin^{R}), cetuximab, Iressa, OSI-774, CI-1033, EKB-569, GW-2016, E1.1, E2.4, E2.5, E6.2, E6.4, E2.11, E6.3 or E7.6.3.

Compounds which target, decrease or inhibit the activity of PDGFR are especially compounds which inhibit the PDGF receptor, e.g. a N-phenyl-2-pyrimidine-amine derivative, e.g. imatinib.

Compounds which target, decrease or inhibit the activity of c-AbI family members and their gene fusion products are, e.g. a N-phenyl-2-pyrimidine-amine derivative, e.g. imatinib; PD180970; AG957; orNSC 680410.

Compounds which target, decrease or inhibit the activity of protein kinase C, Raf, MEK, SRC, JAK, FAK and PDK family members, or PI(3) kinase or PI(3) kinase-related family members, and/or members of the cyclin-dependent kinase family (CDK) are especially those staurosporine derivatives disclosed in EP 0 296 110, e.g. midostaurin; examples of further compounds include e.g. UCN-01, safingol, BAY 43-9006, Bryostatin 1, Perifosine; Ilmofosine; RO 318220 and RO 320432; GO 6976; Isis 3521; or LY333531/LY379196.

Further anti-angiogenic compounds are e.g. thalidomide (THALOMID) and TNP-470.

Compounds which target, decrease or inhibit the activity of a protein or lipid phosphatase are, e.g. inhibitors of phosphatase 1, phosphatase 2A, PTEN or CDC25, e.g. okadaic acid or a derivative thereof.

Compounds which induce cell differentiation processes are, e.g. retinoic acid, α-, γ- or δ-tocopherol or α-, γ- or δ-tocotrienol.

The term cyclooxygenase inhibitor as used herein includes, but is not limited to, e.g. celecoxib (Celebrex^{R}), rofecoxib (Vioxx^{R}), etoricoxib, valdecoxib or a 5-alkyl-2-arylaminophenylacetic acid, e.g. 5-methyl-2-(2'-chloro-6'-fluoroanilino)phenyl acetic acid.

The term "histone deacetylase inhibitor" as used herein includes, but is not limited to MS-27-275, SAHA, pyroxamide, FR-901228 or valproic acid.

The term "bisphosphonates" as used herein includes, but is not limited to, etridonic, clodronic, tiludronic, pamidronic, alendronic, ibandronic, risedronic and zoledronitc acid.

The term "matrix metalloproteinase inhibitor" as used herein includes, but is not limited to collagen peptidomimetic and non-petidomimetic inhibitors, tetracycline derivatives, e.g. hydroxamate peptidomimetic inhibitor batimastat and its orally bioavailable analogue marimastat, prinomastat, BMS-279251, BAY 12-9566, TAA211 or AAJ996.

The term "mTOR inhibitor" as used herein includes, but is not limited to rapamycin (sirolimus) or a derivative thereof, e.g. 32-deoxorapamycin, 16-pent-2-ynyloxy-32-deoxorapamycin, 16-pent-2-ynyloxy-32(S)-dihydro-rapamycin, 16-pent-2-ynyloxy-32(S)-dihydro-40-O-(2-hydroxyethyl)-rapamycin and, more preferably, 40-0-(2-hydroxy-ethyl)-rapamycin. Further examples of rapamycin derivatives include e.g. CCI779 or 40- [3-hydroxy-2-(hydroxymethyl)-2-methylpropanoate]-rapamycin or a pharmaceutically acceptable salt thereof, as disclosed in USP 5,362,718, ABT578 or 40-(tetrazolyl)-rapamycin, particularly 40-epi-(tetrazolyl)-rapamycin, e.g. as disclosed in WO 99/15530, or rapalogs as disclosed e.g. in WO 98/02441 and WO01/14387, e.g. AP23573.

Where the compounds of formula I are administered in conjunction with other immunosuppressive / immunomodulatory, anti-inflammatory or chemotherapeutic therapy, dosages of the co-administered immunosuppressant, immunomodulatory, anti-inflammatory or chemotherapeutic compound will of course vary depending on the type of co-drug employed, e.g. whether it is a steroid or a calcineurin inhibitor, on the specific drug employed, on the condition being treated and so forth.

In accordance with the foregoing the present invention provides compounds of Formula (I) for use in:
5. A method as defined above comprising co-administration, e.g. concomitantly or in sequence, of a therapeutically effective non-toxic amount of a compound of formula I and at least a second drug substance, e.g. an immunosuppressant, immunomodulatory, anti-inflammatory or chemotherapeutic drug, e.g. as indicated above. The invention also provides:
6. A pharmaceutical combination, e.g. a kit, comprising a) a first agent which is a compound of formula I as disclosed herein, in free form or in pharmaceutically acceptable salt form, and b) at least one co-agent, e.g. an immunosuppressant, immunomodulatory, anti-inflammatory or chemotherapeutic drug, e.g. as disclosed above. The kit may comprise instructions for its administration.

The terms "co-administration" or "combined administration" or the like as utilized herein are meant to encompass administration of the selected therapeutic agents to a single patient, and are intended to include treatment regimens in which the agents are not necessarily administered by the same route of administration or at the same time.

The term "pharmaceutical combination" as used herein means a product that results from the mixing or combining of more than one active ingredient and includes both fixed and non-fixed combinations of the active ingredients. The term "fixed combination" means that the active ingredients, e.g. a compound of formula I and a co-agent, are both administered to a patient simultaneously in the form of a single entity or dosage. The term "non-fixed combination" means that the active ingredients, e.g. a compound of formula I and a co-agent, are both administered to a patient as separate entities either simultaneously, concurrently or sequentially with no specific time limits, wherein such administration provides therapeutically effective levels of the 2 compounds in the body of the patient. The latter also applies to cocktail therapy, e.g. the administration of 3 or more active ingredients.

### Methods for Preparing Compounds of the Invention

The present invention also includes processes for the preparation of immunomodulatory compounds of the invention. In the reactions described, it can be necessary to protect reactive functional groups, for example hydroxy, amino, imino, thio or carboxy groups, where these are desired in the final product, to avoid their unwanted participation in the reactions. Conventional protecting groups can be used in accordance with standard practice, for example, see T.W. Greene and P. G. M. Wuts in "Protective Groups in Organic Chemistry", John Wiley and Sons, 1991.

Compounds of Formula I can be prepared by proceeding as in the following reaction scheme: 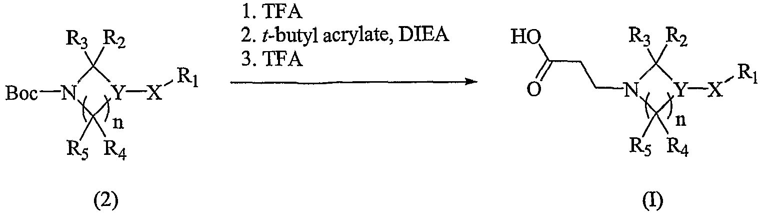 in which n, X, R₁, R₂, R₃, R₄ and R₅ are as defined for Formula I above. Compounds of Formula I can be prepared sequentially by treating a compound of formula 2 with a suitable acid (e.g. TFA, and the like), reacting with *t*-butyl acrylate in the presence of a suitable amine (e.g. DIEA, and the like) and removing the *t*-butyl protecting group with a suitable acid (e.g. TFA, and the like). The reaction proceeds at a temperature of about 0 to about 120°C and can take up to about 24 hours to complete.

### Additional Processes for Preparing Compounds of the Invention:

A compound of the invention can be prepared as a pharmaceutically acceptable acid addition salt by reacting the free base form of the compound with a pharmaceutically acceptable inorganic or organic acid. Alternatively, a pharmaceutically acceptable base addition salt of a compound of the invention can be prepared by reacting the free acid form of the compound with a pharmaceutically acceptable inorganic or organic base. Alternatively, the salt forms of the compounds of the invention can be prepared using salts of the starting materials or intermediates.

The free acid or free base forms of the compounds of the invention can be prepared from the corresponding base addition salt or acid addition salt from, respectively. For example a compound of the invention in an acid addition salt form can be converted to the corresponding free base by treating with a suitable base (e.g., ammonium hydroxide solution, sodium hydroxide, and the like). A compound of the invention in a base addition salt form can be converted to the corresponding free acid by treating with a suitable acid (e.g., hydrochloric acid, etc.).

Compounds of the invention in unoxidized form can be prepared from N-oxides of compounds of the invention by treating with a reducing agent (e.g., sulfur, sulfur dioxide, triphenyl phosphine, lithium borohydride, sodium borohydride, phosphorus trichloride, tribromide, or the like) in a suitable inert organic solvent (e.g. acetonitrile, ethanol, aqueous dioxane, or the like) at 0 to 80°C.

Prodrug derivatives of the compounds of the invention can be prepared by methods known to those of ordinary skill in the art (e.g., for further details see Saulnier et al., (1994), Bioorganic and Medicinal Chemistry Letters, Vol. 4, p. 1985). For example, appropriate prodrugs can be prepared by reacting a non-derivatized compound of the invention with a suitable carbamylating agent (e.g., 1,1-acyloxyalkylcarbanochloridate, para-nitrophenyl carbonate, or the like).

Protected derivatives of the compounds of the invention can be made by means known to those of ordinary skill in the art. A detailed description of techniques applicable to the creation of protecting groups and their removal can be found in T W. Greene, "Protecting Groups in Organic Chemistry", 3rd edition, John Wiley and Sons, Inc., 1999.

Compounds of the present invention can be conveniently prepared, or formed during the process of the invention, as solvates (e.g., hydrates). Hydrates of compounds of the present invention can be conveniently prepared by recrystallization from an aqueous/organic solvent mixture, using organic solvents such as dioxin, tetrahydrofuran or methanol.

Compounds of the invention can be prepared as their individual stereoisomers by reacting a racemic mixture of the compound with an optically active resolving agent to forma pair of diastereoisomeric compounds, separating the diastereomers and recovering the optically pure enantiomers. While resolution of enantiomers can be carried out using covalent diastereomeric derivatives of the compounds of the invention, dissociable complexes are preferred (e.g., crystalline diastereomeric salts). Diastereomers have distinct physical properties (e.g., melting points, boiling points, solubilities, reactivity, etc.) and can be readily separated by taking advantage of these dissimilarities. The diastereomers can be separated by chromatography, or preferable, by separation/resolution techniques based upon differences in solubility. The optically pure enantiomer is then recovered, along with the resolving agent, by any practical means that would not result in racemization. A more detailed description of the techniques applicable to the resolution of stereoisomers of compounds from the their racemic mixture can be found in Jean Jacques, Andre Collet, Samuel H. Wilen, "Enantiomers, Racemates and Resolutions", John Wiley And Sons, Inc., 1981.

In summary, the compounds of Formula I can be made by a process, which involves:
(a) the above reaction scheme; and
(b) optionally converting a compound of the invention into a pharmaceutically acceptable salt;
(c) optionally converting a salt form of a compound of the invention to a non-salt form;
(d) optionally converting an unoxidized form of a compound of the invention into a pharmaceutically acceptable N-oxide;
(e) optionally converting an N-oxide form of a compound of the invention to its unoxidized form;
(f) optionally resolving an individual isomer of a compound of the invention from a mixture of isomers;
(g) optionally converting a non-derivatized compound of the invention into a pharmaceutically acceptable prodrug derivative; and
(h) optionally converting a prodrug derivative of a compound of the invention to its non-derivatized form.

Insofar as the production of the starting materials is not particularly described, the compounds are known or can be prepared analogously to methods known in the art or as disclosed in the Examples hereinafter.

One of skill in the art will appreciate that the above transformations are only representative of methods for preparation of the compounds of the present invention, and that other well known methods can similarly be used.

### EXAMPLES

The following examples provide detailed descriptions of the preparation of representative compounds and are offered to illustrate, but not to limit the present invention.

### Example 1

### Synthesis of 3-{6-[3-(2-Trifluoromethyl-biphenyl-4-yl)-[1,2,4]oxadiazol-5-yl]-3,4-dihydro-1H-isoquinolin-2-yl}-propionic acid

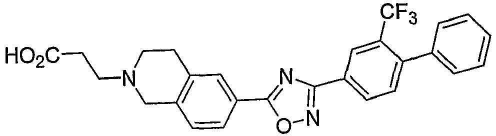

To a suspension of 3,4-dihydro-1H-isoquinoline-2,6-dicarboxylic acid 2-tert-butyl ester (96 mg, 0.348 mmol) in toluene (4 mL) is added SOCl₂ (254 µL, 10 eq.). The mixture is heated to reflux for 3 hours. All the solvent is removed under reduced pressure. The residue is redissolved in toluene and evaporated to dryness twice to remove excess HCl and is dried under high vacuum for 2 hours to give crude 6-chlorocarbonyl-3,4-dihydro-1H-isoquinoline-2-carboxylic acid tert-butyl ester.

In a separate flask is charged with N-hydroxy-2-trifluoromethyl-biphenyl-4-carboxamidine (82 mg, 0.29 mmol) and DIEA (242 µL, 4 eq.) and CH₂Cl₂ (5 mL). The mixture is cooled to 0°C using an ice-salt bath. The chloride from the previous step is dissolved in CH₂Cl₂ (2 mL) and added slowly. After addition, the resulting mixture is warmed to room temperature and is stirred for 2 hours. All the solvent is evaporated and the mixture is purified by column chromatography (silica gel, EtOAc/Hexane, gradient) to 120 mg of the desired product. The product is dissolved in THF (2 mL) and mixed with TBAF (444 µL, 2 eq.) in a microwave vial. The mixture is heated to 100°C for 15 minutes using microwave irradiation. All the solvent is evaporated and the residue is purified by column chromatography (silica gel, EtOAc/Hexane, gradient) to give 70 mg of 6-[3-(2-trifluoromethyl-biphenyl-4-yl)-[1,2,4]oxadiazol-5-yl]-3,4-dihydro-1H-isoquinoline-2-carboxylic acid tert-butyl ester.

To a solution of 6-[3-(2-trifluoromethyl-biphenyl-4-yl)-[1,2,4]oxadiazol-5-yl]-3,4-dihydro-1H-isoquinoline-2-carboxylic acid tert-butyl ester (68 mg, 0.13 mmol) in CH₂Cl₂ (1 mL) is added TFA(2 mL). The mixture is stirred at room temperature for 30 minutes. All the solvents are removed under reduced pressure. The mixture is dissolved in CH₃OH (1.5 mL). Then DIEA (119 µL, 10 eq.) and acrylic acid tert-butyl ester (38 µL, 2 eq.) are added. The mixture is heated to 90°C for 20 minutes using microwave irradiation. All the solvent is evaporated and the crude product of 3-{6-[3-(2-trifluoromethyl-biphenyl-4-yl)-[1,2,4]oxadiazol-5-yl]-3,4-dihydro-1H-isoquinolin-2-yl}-propionic acid tert-butyl ester is used in the next step without further purification.

To a solution of crude 3-{6-[3-(2-trifluoromethyl-biphenyl-4-yl)-[1,2,4]oxadiazol-5-yl]-3,4-dihydro-1H-isoquinolin-2-yl}-propionic acid tert-butyl ester in CH₂Cl₂ (1 mL) is added TFA(1 mL). The mixture is stirred at room temperature for an hour. All the solvents are evaporated. The mixture is purified by reverse phase preparative LC/MS to give 3- {6-[3-(2-Trifluoromethyl-biphenyl-4-yl)-[1,2,4]oxadiazol-5-yl]-3,4-dihydro-1H-isoquinolin-2-yl}-propionic acid: ¹H NMR (DMSO, 400 MHz) δ 8.52 (s, 1H), 8.38 (d, 1H), 8.15 (s, 1H), 8.10 (d, 1H), 7.68 (d, 1H), 7.50 (m, 4H), 7.38 (m, 2H), 3.20-3.50 (m, 8H), 2.92 (t, 2H), MS (ES⁺): (494.10, M+1)⁺.

### Example 2

### Synthesis of 3-[6-(2-Trifluoromethyl-biphenyl-4-yloxmethyl)-3,4-dihydro-1H-isoquinolin-2-yl]-propionic acid

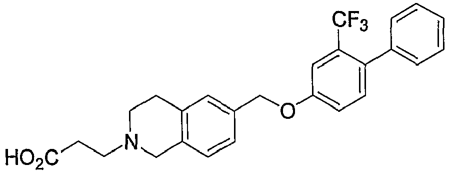

To a solution of 6-hydroxymethyl-3,4-dihydro-1H-isoquinoline-2-carboxylic acid tert-butyl ester (85 mg, 0.323 mmol) in CH₂Cl₂ (1.5 mL) is added a solution of 4-chloro-3-trifluoromethyl-phenol (76 mg, 1.23 eq.) in CH₂Cl₂ (0.5 mL), PPh₃ (127 mg, 1.5 eq.), and 1,1'-(azodicarbonyl)-dipiperidine (122 mg, 1.5 eq.). The mixture is stirred at room temperature overnight. All the solvent is removed under reduced pressure and the mixture is purified by column chromatography (silica gel, EtOAc/Hexane, gradient) to give 70 mg of 6-(4-chloro-3-trifluoromethyl-phenoxymethyl)-3,4-dihydro-1H-isoquinoline-2-carboxylic acid tert-butyl ester

To a solution of 6-(4-chloro-3-trifluoromethyl-phenoxymethyl)-3,4-dihydro-1H-isoquinoline-2-carboxylic acid tert-butyl ester (70 mg, 0.158 mmol) in CH₂Cl₂ (1 mL) is added TFA(1 mL). The mixture is stirred at room temperature for 30 minutes. All the solvents are removed under reduced pressure. The mixture is dissolved in CH₃OH (1.5 mL). Then DIEA (269 µL, 10 eq.) and acrylic acid tert-butyl ester (46 µL, 2 eq.) are added. The mixture is heated to 90°C for 20 minutes using microwave irradiation. All the solvent is evaporated and the mixture is purified by column chromatography (silica gel, EtOAc/Hexane, gradient) to give 56 mg of 3-[6-(4-chloro-3-trifluoromethyl-phenoxymethyl)-3,4-dihydro-1H-isoquinolin-2-yl]-propionic acid tert-butyl ester.

A microwave vial is charged with 3-[6-(4-chloro-3-trifluoromethyl-phenoxymethyl)-3,4-dihydro-1H-isoquinolin-2-yl]-propionic acid tert-butyl ester (56 mg, 0.119 mmol), phenylboronic acid (17 mg, 1.2 eq.), Pd(OAc)₂ (1.3 mg, 5 mol%), (dicyclohexylphosphino)biphenyl (4.2mg, 10 mol%), KF (21mg, 3 eq.), and THF (0.25 mL). The resulting mixture is heated to 120°C using microwave irradiation for 20 minutes. The mixture is filtered through celite. The celite is washed with EtOAc several times. The filtrate is then concentrated to give a dark oil. The mixture is purified by column chromatography (silica gel, EtOAc/Hexane, gradient) to give 3-[6-(2-trifluoromethyl-biphenyl-4-yloxymethyl)-3,4-dihydro-1H-isoquinolin-2-yl]-propionic acid tert-butyl ester.

To a solution of 3-[6-(2-trifluoromethyl-biphenyl-4-yloxymethyl)-3,4-dihydro-1H-isoquinolin-2-yl]-propionic acid tert-butyl ester (35 mg, 0.068 mmol) in CH₂Cl₂ (1 mL) is added TFA (1 mL). The mixture is stirred at room temperature for an hour. All the solvents are evaporated. The mixture is purified by reverse phase preparative LC/MS to give 3-[6-(2-trifluoromethyl-biphenyl-4-yloxymethyl)-3,4-dihydro-1H-isoquinolin-2-yl]-propionic acid: ¹H NMR (CD₃OD, 400 MHz) δ 7.45 (m, 2H), 7.38-7.41 (m, 3H), 7.35 (s, 1H), 7.16-7.23 (m, 5H), 4.85 (s, 2H), 4.54 (s, 2H), 3.68 (m, 2H), 3.60 (t, 2H), 3.27 (t, 2H), 2.96 (t, 2H), MS (ES⁺): (456.20, M+1)⁺.

By repeating the procedure described in the above examples, using appropriate starting materials, the following compounds of Formula I are obtained as identified in Table 1.

**TABLE 1**

| **Compound** | **Structure** | **Physical Data MS ES (M+1)** |
|---|---|---|
| 1 | 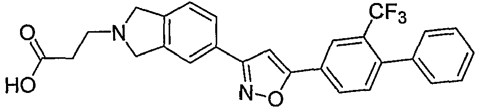 | 479.2 |
| 2 | 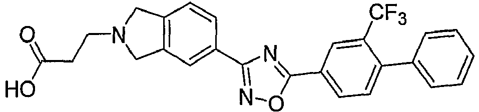 | 480.2 |
| 3 | 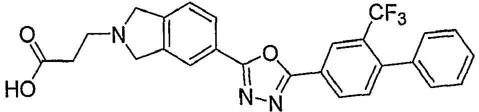 | 480.2 |
| 4 | 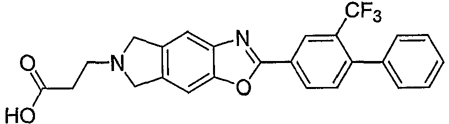 | 453.1 |
| 5 | 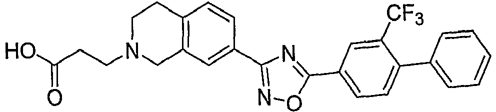 | 494.2 |
| 6 | 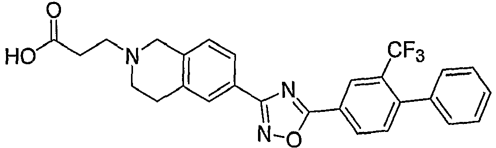 | 494.2 |
| 7 | 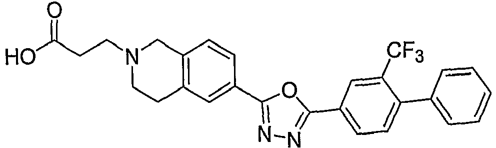 | 494.2 |
| 8 | 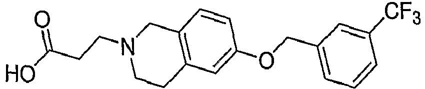 | 380.1 |
| 9 | 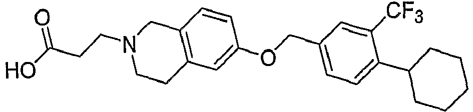 | 462.2 |
| 10 | 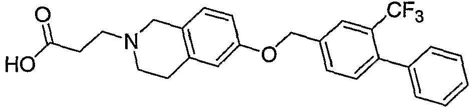 | 456.2 |
| 11 | 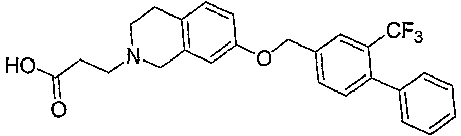 | 456.2 |
| 12 | 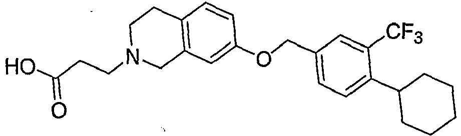 | 462.3 |
| 13 | 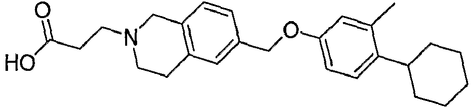 | |
| 14 | 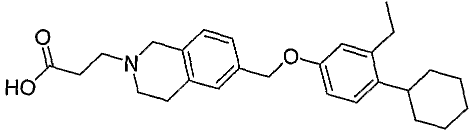 | |
| 15 | 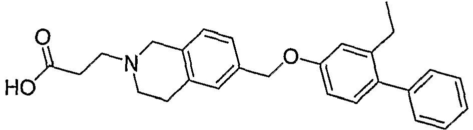 | |
| 16 | 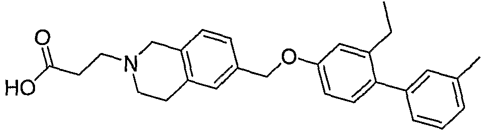 | |

### Example 3

### Compounds of Formula I Exhibit Biological Activity

### A. In vitro: GPCR activation assay measuring GTP [γ-³⁵S] binding to membranes prepared from CHO cells expressing human EDG receptors

EDG-1 (SIP₁) GTP [γ-³⁵S] binding assay: Homogenized membranes are prepared from CHO cell clones stably expressing a human EDG-1 N-terminal c-myc tag. Cells are grown in suspension in two 850 cm² roller bottles for three or fours days before harvesting. The cells are centrifuged down, washed once with cold PBS, and resuspended in ≤20 ml of Buffer A (20 mM HEPES, pH 7.4, 10 mM EDTA, EDTA-free complete protease inhibitor cocktail [1 tablet/25 ml]). The cell suspension is homogenized on ice, using a Polytron homogenizer at 30000 rpm at three intervals of 15 seconds each. The homogenate is first centrifuged at 2000 rpm on a tabletop low speed centrifuge for 10 minutes. The supernatant, after passing through a cell strainer, is then re-centrifuged at 50,000 x g for 25 minutes at 4°C. The pellet is resuspended into buffer B (15% glycerol, 20 mM HEPES, pH 7.4, 0.1 mM EDTA, EDTA-free complete protease inhibitor cocktail [1 tablet/10 ml]). Protein concentration of the prep is determined using the BCA Protein Assay kit (Pierce) using BSA as standard. The membranes are aliquoted and kept frozen at -80°C.

Solutions of test compounds ranging from 10mM to 0.01nM are prepared in DMSO. S1P is diluted in 4% BSA solution as positive controls. The desired amount of membrane prep is diluted with ice-cold assay buffer (20 mM HEPES, pH 7.4, 100 mM NaCl, 10 mM MgCl₂, 0.1% Fatty acid-free BSA, 5 µM GDP) and vortexed well. 2 µl or less of compound is distributed into each well of a round-bottom 96-well polystyrene assay plate, followed by addition of 100 µl of diluted membranes (3-10 µg/well) and kept on ice until the addition of hot GTPγS. [³⁵S]-GTPγS is diluted 1:1000 (v/v) with cold assay buffer and 100 γl is added into each well. The reaction is carried out at room temperature for 90 minutes before the membranes are harvested onto Perkin-Elmer Unifilter^{®} GF/B-96 filter plate using a Packard Filtermate Harvester. After several washes with wash buffer (20 mM HEPES, pH 7.4, 100 mM NaCl, 10 mM MgCl₂), and a rinse with 95% ethanol, the filter is dried in a 37°C oven for 30 minutes. MicroScint-20 is added and the plate sealed for scintillation counting on TopCount. EC50 values are obtained by fitting the GTP [γ-³⁵S] binding curves (raw data) with the dose response curve-fitting tool of GraphPad Prism. Six or twelve different concentrations are used to generate a concentration response curve (using three data points per concentration).

EDG-3,-5,-6 and -8 GTP [γ-³⁵S] binding assays are carried out in a comparable manner to the EDG-1 GTP [γ-³⁵S] binding assay using membranes from CHO cells stably expressing c-terminal c-myc tagged or untagged receptors. For each membrane preparation, titration experiments are first run with S1P control to determine the optimal amount of membranes to be added per assay well. Compounds of the invention were tested according to the above assay and were observed to exhibit selectivity for the EDG-1 receptor. For example, 3-[6-(4-cyclohexyl-3-trifluoromethyl-benzyloxy)-3,4-dihydro-1H-isoquinolin-2-yl]-propionic acid (compound 9) has an EC₅₀ of 0.2 nM in the above assay and is at least 1000 fold selective for EDG-1 compared to one or more of the other receptors including EDG-3, EDG-5, EDG-6 and EDG-8.

### B. In vitro: FLIPR calcium flux assay

Compounds of the invention are tested for agonist activity on EDG-1, EDG-3, EDG-5, and EDG-6 with a FLIPR calcium flux assay. Briefly, CHO cells expressing an EDG receptor are maintained in F-12K medium (ATCC), containing 5% FBS, with 500ug/ml of G418. Prior to the assay, the cells are plated in 384 black clear bottom plates at the density of 10,000 cells/well/25µl in the medium of F-12K containing 1% FBS. The second day, the cells are washed three times (25 µl/each) with washing buffer. About 25 µl of dye are added to each well and incubated for 1 hour at 37°C and 5% CO₂. The cells are then washed four times with washing buffer (25 µl/each). The calcium flux is assayed after adding 25 µl of SEQ2871 solution to each well of cells. The same assay is performed with cells expressing each of the different EDG receptors. Titration in the FLIPR calcium flux assay is recorded over a 3-minute interval, and quantitated as maximal peak height percentage response relative to EDG-1 activation.

### C. In vivo: Screening Assays for measurement of blood lymphocyte depletion and assessment of heart effect

Measurement of circulating lymphocytes: Compounds are dissolved in DMSO and diluted to obtain a final concentration of 4% DMSO (v/v, final concentration) and then further diluted in a constant volume of Tween80 25%/H2O, v/v. Tween80 25%/H2O (200 µl), 4% DMSO, and FTY720 (10µg) are included as negative and positive controls, respectively. Mice (C57bl/6 male, 6-10 week-old) are administered 250-300 µL of compound solution orally by gavages under short isoflurane anesthesia.

Blood is collected from the retro-orbital sinus 6 and 24 hours after drug administration under short isoflurane anesthesia. Whole blood samples are subjected to hematology analysis. Peripheral lymphocyte counts are determined using an automated analyzer. Subpopulations of peripheral blood lymphocytes are stained by fluorochrome-conjugated specific antibodies and analyzed using a fluorescent activating cell sorter (Facscalibur). Two mice are used to assess the lymphocyte depletion activity of each compound screened. The result is an ED₅₀, which is defined as the effective dose required displaying 50 % of blood lymphocyte depletion. Compounds of the invention were tested according to the above assay and were preferably found to exhibit an ED₅₀ of less than 1mg/kg, more preferably an ED₅₀ of less than 0.5 mg/kg. For example, 3-[6-(4-cyclohexyl-3-trifluoromethyl-benzyloxy)-3,4-dihydro-1H-isoquinolin-2-yl]-propionic acid (compound 9) exhibits an ED50 of 0.2 mg/kg.

Assessment of Heart Effect: The effects of compounds on cardiac function are monitored using the AnonyMOUSE ECG screening system. Electrocardiograms are recorded in conscious mice (C57bl/6 male, 6-10 week-old) before and after compound administration. ECG signals are then processed and analyzed using the e-MOUSE software. 90 µg of compound further diluted in 200µl water, 15% DMSO are injected IP. Four mice are used to assess the heart effect of each compound.

### D: In vivo: Anti-angiogenic Activity

Porous chambers containing (i) sphingosine-1-phosphate (5 µM/chamber) or (ii) human VEGF (1 µg/chamber) in 0.5 ml of 0.8% w/v agar (containing heparin, 20 U/ml) are implanted subcutaneously in the flank of mice. S1P or VEGF induces the growth of vascularized tissue around the chamber. This response is dose-dependent and can be quantified by measuring the weight and blood content of the tissue. Mice are treated once a day orally or intravenously with a compound of formula I starting 4-6 hours before implantation of the chambers and continuing for 4 days. The animals are sacrificed for measurement of the vascularized tissues 24 hours after the last dose. The weight and blood content of the vascularized tissues around the chamber is determined. Animals treated with a compound of formula I show reduced weight and/or blood content of the vascularized tissues compared to animals treated with vehicle alone. Compounds of Formula I are anti-angiogenic when administered at a dose of about 0.3 to about 3mg/kg.

### E: In vitro: Antitumor Activity

A mouse breast cancer cell line originally isolated from mammary carcinomas is used, e.g. JygMC(A). The cell number is adjusted to 5x10⁵ for plating in fresh medium before the procedure. Cells are incubated with fresh medium containing 2.5mM of thymidine without FCS for 12 hours and then washed twice with PBS, followed by addition of fresh medium with 10% FCS and additionally incubated for another 12 hours. Thereafter the cells are incubated with fresh medium containing 2.5mM of thymidine without FCS for 12 hours. To release the cells from the block, the cells are washed twice with PBS and replated in fresh medium with 10% FCS. After synchronization, the cells are incubated with or without various concentrations of a compound of formula I for 3, 6, 9, 12, 18 or 24 hours. The cells are harvested after treatment with 0.2% EDTA, fixed with ice-cold 70% ethanol solution, hydrolyzed with 250□g/ml of RNaseA (type 1-A: Sigma Chem. Co.) at 37°C for 30 minutes and stained with propidium iodide at 10mg/ml for 20 minutes. After the incubation period, the number of cells is determined both by counting cells in a Coulter counter and by the SRB colorimetric assay. Under these conditions compounds of formula I inhibit the proliferation of the tumor cells at concentrations ranging from 10⁻¹² to 10⁻⁶ M.

## Claims

1. A compound of Formula I: 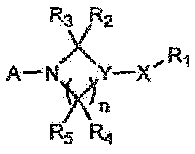 in which:
n is 1 or 2;
A is -(CH₂)₂C(O)OH;
X is a bond or is selected from [1,2,4]oxadiazole, -CH₂O-, -OCH₂-, isoxazoles and [1,3,4]oxadiazole;
Y is selected from phenyl and benzooxazolyl;
R₁ is chosen from C₆₋₁₀aryl and C₂₋₉heteroaryl; wherein any aryl or heteroaryl of R₁ is optionally substituted by a radical chosen from C₆₋₁₀arylC₀₋₄alkyl, C₂₋₉heteroaxylC₀-₄alkyl, C₃₋₈cycloalkylC₀₋₄alkyl, C₃₋₈heterocycloalkylC₀₋₄alkyl or C₁₋₆alkyl; wherein any aryl, heteroaryl, cycloalkyl or heterocycloalkyl group of R₁ can be optionally substituted by one to five radicals chosen from halo, C₁₋₆alkyl, C₁₋₆alkoxy, halo-substituted-C₁₋₆alkyl and halo-substituted-C₁₋₆alkoxy; and any alkyl group of R₁ can optionally have a methylene replaced by an atom or group chosen from -S-, -S(O)-, -S(O)₂-, -NR₇- an -O-; wherein R₇ is chosen from hydrogen or C₁₋₆alkyl;
R₂, R₃, R₄ and R₅ are hydrogen; and the pharmaceutically acceptable salts, hydrates, solvates, N-oxide derivatives, optical or geometric isomers selected from cis-compounds, trans compounds and mixtures thereof.

2. The compound of claim 1 in which R₁ is phenyl, naphthyl, furanyl or thienyl optionally substituted by C₆₋₁₀arylC₀₋₄alkyl, C₂₋₉heteroarylC₀₋₄alkyl, C₃₋₈cycloalkylC₀₋₄alkyl, C₃₋₈heterocycloalkylC₀₋₄alkyl or C₁₋₆alkyl; wherein any aryl, heteroaryl, cycloalkyl or heterocycloalkyl group of R₁ can be optionally substituted by one to five radicals chosen from halo, C₁₋₆alkyl, C₁₋₆alkoxy, halo-substituted-C₁₋₆alkyl and halo-substituted-C₁₋₆alkoxy; and any alkyl group of R₁ can optionally have a methylene replaced by an atom or group chosen from -S-, -S(O)-, -S(O)₂-, -NR₇- and -O-; wherein R₇ is hydrogen or C₁₋₆alkyl.

3. The compound of claim 1 in which R₁ is chosen from: 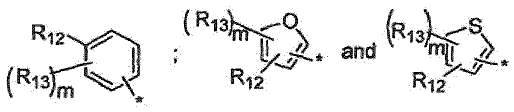 wherein the asterisk is the point of attachment of R₁ with X; m is chosen from 1 and 2; R₁₂ is selected from hydrogen, C₆₋₁₀arylC₀₋₄alkyl, C₂₋₉heteroarylC₀₋₄alkyl, C₃₋₈cycloalkylC₀₋₄alkyl, C₃₋₈heterocycloalkylC₀₋₄alkyl or C₁₋₆alkyl; wherein any aryl, heteroaryl, cycloalkyl or heterocycloalkyl group of R₁₂ can be optionally substituted by one to three radicals chosen from halo, C₁₋₆alkyl, C₁₋₆alkoxy, halo-substituted-C₁₋₆alkyl and halo-substituted-C₁₋₆alkoxy; and any alkyl group of R₁₂ can optionally have a methylene replaced by an atom or group chosen from -S-, -S(O)-, -S(O)₂-, -NR₁₀- and -O-; wherein R₁₀ is hydrogen or C₁₋₆alkyl; and R₁₃ is chosen from halo, C₁₋₆alkyl, C₁₋₆alkoxy, halo-substituted-C₁₋₆alkyl and halo-substituted-C₁₋₆alkoxy.

4. The compound of claim 1 in which R₁ is selected from: 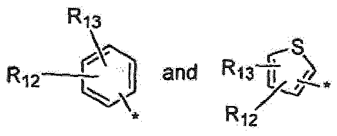 wherein R₁₂ is selected from hydrogen, phenyl and cyclohexyl; wherein any phenyl or cyclohexyl of R₁₂ is optionally substituted with methyl; and R₁₃ is selected from trifluoromethyl, methyl and ethyl.

5. The compound of claim 4 selected from 3-{6-[3-(2-trifluoromethyl-biphenyl-4-yl)-[1,2,4]oxadiazol-5-yl]-3,4-dihydro-1H-isoquinolin-2-yl}-propionic acid, 3-[6-(2-trifluoromethyl-biphenyl-4-yloxymethyl)-3,4-dihydro-1H-isoquinolin-2-yl]-propionic acid, 3-{5-[5-(2-trifluoromethyl-biphenyl-4-yl)-isoxazol-3-yl]-1,3-dihydro-isoindol-2-yl}-propionic acid, 3-{5-[5-(2-trifluoromethyl-biphenyl-4-yl)-[1,2,4]oxadiazol-3-yl]-1,3-dihydro-isoindol-2-yl}-propionic acid, 3-{5-[5-(2-trifluoromethyl-biphenyl-4-yl)-[1,3,4]oxadiazol-2-yl]-1,3-dihydro-isoindol-2-yl}-propionic acid, 3-[2-(2-trifluoromethyl-biphenyl-4-yl)-5,7-dihydro-oxazolo[4,5-f]isoindol-6-yl]-propionic acid, 3-{7-[5-(2-trifluoromethyl-biphenyl-4-yl)-[1,2,4]oxadiazol-3-yl]-3,4-dihydro-1H-isoquinolin-2-yl}-propionic acid, 3-{6-[5-(2-trifluoromethyl-biphenyl-4-yl)-[1,2,4]oxadiazol-3-yl]-3,4-dihydro-1H-isoqninolin-2-yl}-propionic acid, 3-{6-[5-(2-trifluoromethyl-biphenyl-4-yl)-[1,3,4]oxadiazol-2-yl]-3,4-dihydro-1H-isoquinolin-2-yl}-propionic acid, 3-[6-(3-trifluoromethyl-benzyloxy)-3,4-dihydro-1H-isoquinolin-2-yl]-propionic acid, 3-[6-(4-cyclohexyl-3-trifluoromethyl-benzyloxy)-3,4-dihydro-1H-isoquinolin-2-yl]-propionic acid, 3-[6-(2-trifluoromethyl-biphenyl-4-ylmethoxy)-3,4-dihydro-1H-isoquinolin-2-yl]-propionic acid, 3- [7-(2- trifluoromethyl-biphenyl-4 ylmethoxy)-3,4 -dihydro-1H-isoquinolin-2-yl]-propionil acid 3-[7-(4-cyclohexyl-3-trifluoromethyl-benzyloxy)-3,4-dihydro-1H-isoquinolin-2-yl]-propionic acid, 3-[6-(4-cyclohexyl-3-methyl-phenoxymethyl)-3,4-dihydro-1H-isoquinolin-2-yl]-propionic acid, 3-[6-(4-cyclohexyl-3-ethyl-phenoxymethyl)-3,4-dihydro-1H-isoquinolin-2-yl]-propionic acid, 3-[6-(2-ethyl-biphenyl-4-yloxymethyl)-3,4-dihydro-1H-isoquinolin-2-yl]-propionic acid and 3-[6-(2-ethyl-3'-methyl-biphenyl-4-yloxymethyl)-3,4-dihydro-1H-isoquinolin-2-yl]-propionic acid.

6. A pharmaceutical composition comprising a therapeutically effective amount of a compound of Claim 1 in combination with a pharmaceutically acceptable excipient.

7. The pharmaceutical composition of claim 6, comprising a therapeutically effective amount of a compound of any one of claims 1-5, in combination with an immunosuppressive agent, an immunomodulating agent or other anti-inflammatory agent.

8. The pharmaceutical composition of claim 6, comprising a therapeutically effective amount of a compound of any one of claims 1-5, in combination with one or more of:
i. an aromatase inhibitor,
ii. an anti-estrogen, an anti-androgen (especially in the case of prostate cancer) or a gonadorelin agonist,
iii. a topoisomerase I inhibitor or a topoisomerase II inhibitor,
iv. a microtubule active agent, an alkylating agent, an antineoplastic antimetabolite or a platin compound,
v. a compound targeting/decreasing a protein or lipid kinase activity or a protein or lipid phosphatase activity, a further anti-angiogenic compound or a compound which induces cell differentiation processes,
vi. a bradykinin 1 receptor or an angiotensin II antagonist,
vii. a cyclooxygenase inhibitor, a bisphosphonate, a histone deacetylase inhibitor, a heparanase inhibitor (prevents heparan sulphate degradation), e. g. PI-88, a biological response modifier, preferably a lymphokine or interferons, a ubiquitination inhibitor, or an inhibitor which blocks anti-apoptotic pathways,
viii. an inhibitor of Ras oncogenic isoforms, e. g. H-Ras, K-Ras or N-Ras, or a farnesyl transferase inhibitor, e. g. L-744,832 or DK8G557,
ix. a telomerase inhibitor, e. g. telomestatin,
x. a protease inhibitor, a matrix metalloproteinase inhibitor, a methionine aminopeptidase inhibitor, e. g. bengamide or a derivative thereof, or a proteosome inhibitor, e. g. PS-341, and/or
xi. a mTOR inhibitor.

9. A compound as claimed in any one of claims 1 to 5 or a composition as claimed in claims 6-8 for use in preventing or treating disorders or diseases mediated by lymphocytes, for preventing or treating acute or chronic transplant rejection or T-cell mediated inflammatory or autoimmune diseases, for inhibiting or controlling deregulated angiogenesis, or for preventing or treating diseases mediated by a neo-angiogenesis process or associated with deregulated angiogenesis in a subject comprising administering to the subject in need thereof an effective amount of a compound of claims 1, or a pharmaceutically acceptable salt thereof.

10. The compound or composition of claim 9, wherein the disease or disorder is selected from transplantation rejection, delayed graft function, graft versus host disease, rheumatoid arthritis, systemic lupus erythematosus, hashimoto's thyroidis, multiple sclerosis, myasthenia gravis, diabetes type I or II and the disorders associated therewith, vasculitis, pernicious anemia, Sjoegren syndrome, uveitis, psoriasis, Graves ophthalmopathy, alopecia areata and others, allergic diseases, inflammatory bowel disease, Crohn's disease, ulcerative colitis, intrinsic asthma, inflammatory lung injury, inflammatory liver injury, inflammatory glomerular injury, atherosclerosis, osteoarthritis, irritant contact dermatitis, eczematous dermatitises, seborrhoeic dermatitis, cutaneous manifestations of immunologically-mediated disorders, inflammatory eye disease, keratoconjunctivitis, myocarditis, hepatitis, ischemia/reperfusion injury, stroke, gut ischemia, renal failure, hemorrhage shock, traumatic shock, T cell lymphomas, T cell leukemias, infectious diseases and senile dementia.

## Patentansprüche

1. Verbindung der Formel I: 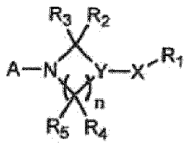 wobei:
n 1 oder 2 ist;
A -(CH₂)₂C(O)OH ist;
X eine Bindung ist oder ausgewählt ist aus [1,2,4]Oxadiazol, -CH₂O-, -OCH₂-, Isoxazolen und [1,3,4]Oxadiazol;
Y ausgewählt ist aus Phenyl und Benzooxazolyl;
R₁ ausgewählt ist aus C₆₋₁₀-Aryl und C₂₋₉-Heteroaryl; wobei jedes Aryl oder Heteroaryl von R₁ optional mit einem Rest ausgewählt aus C₆₋₁₀-Aryl-C₀₋₄-alkyl, C₂₋₉-Heteroaryl-C₀₋₄-alkyl, C₃₋₈-Cycloalkyl-C₀₋₄-alkyl, C₃₋₈-Heterocycloalkyl-C₀₋₄-alkyl oder C₁₋₆-Alkyl substituiert ist; wobei jede Aryl-, Heteroaryl-, Cycloalkyl- oder Heterocycloalkylgruppe von R₁ optional mit einem bis fünf Resten ausgewählt aus Halogen, C₁₋₆-Alkyl, C₁₋₆-Alkoxy, Halogen-substituiertem C₁₋₆-Alkyl und Halogen-substituiertem C₁₋₆-Alkoxy substituiert sein kann; und bei einer beliebigen Alkylgruppe von R₁ optional eine Methylen durch ein Atom oder eine Gruppe ausgewählt aus -S-, -S(O)-, -S(O)₂-, -NR₇- und -0-ersetzt sein kann; wobei R₇ ausgewählt ist aus Wasserstoff oder C₁₋₆-Alkyl;
R₂, R₃, R₄ und R₅ Wasserstoff sind; und die pharmazeutisch annehmbaren Salze, Hydrate, Solvate, N-Oxidderivate, optische oder geometrische Isomere ausgewählt aus cis-Verbindungen, trans-Verbindungen und Mischen davon.

2. Verbindung nach Anspruch 1, wobei R₁ Phenyl, Naphthyl, Furanyl oder Thienyl ist, optional substituiert mit C₆₋₁₀-Aryl-C₀₋₄-alkyl, C₂₋₉-Heteroaryl-C₀₋₄-alkyl, C₃₋₈-Cycloalkyl-C₀₋₄-alkyl, C₃₋₈-Heterocycloalkyl-C₀₋₄-alkyl oder C₁₋₆-Alkyl; wobei jede Aryl-, Heteroaryl-, Cycloalkyl- oder Heterocycloalkylgruppe von R₁ optional mit einem bis fünf Resten ausgewählt aus Halogen, C₁₋₆-Alkyl, C₁₋₆-Alkoxy, Halogen-substituiertem C₁₋₆-Alkyl und Halogen-substituiertem C₁₋₆-Alkoxy substituiert sein kann; und bei einer beliebigen Alkylgruppe von R₁ optional eine Methylen durch ein Atom oder eine Gruppe ausgewählt aus -S-, -S(O)-, -S(O)₂-, -NR₇- und -0- ersetzt sein kann; wobei R₇ Wasserstoff oder C₁₋₆-Alkyl ist.

3. Verbindung nach Anspruch 1, wobei R₁ ausgewählt ist aus: 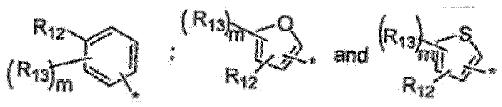 wobei das Sternchen der Verbindungspunkt von R₁ mit X ist; m aus 1 und 2 ausgewählt ist; R₁₂ ausgewählt ist aus Wasserstoff, C₆₋₁₀-Aryl-C₀₋₄-alkyl, C₂₋₉-Heteroaryl-C₀₋₄-alkyl, C₃₋₈-Cycloalkyl-C₀₋₄-alkyl, C₃₋₈-Heterocycloalkyl-C₀₋₄-alkyl oder C₁₋₆-Alkyl; wobei jede Aryl-, Heteroaryl-, Cycloalkyl- oder Heterocycloalkylgruppe von R₁₂ optional mit einem bis drei Resten ausgewählt aus Halogen, C₁₋₆-Alkyl, C₁₋₆-Alkoxy, Halogen-substituiertem C₁₋₆-Alkyl und Halogen-substituiertem C₁₋₆-Alkoxy substituiert sein kann; und bei einer beliebigen Alkylgruppe von R₁₂ optional eine Methylen durch ein Atom oder eine Gruppe ausgewählt aus -S-, - S(O)-, -S(O)₂-, -NR₁₀- und -0- ersetzt sein kann; wobei R₁₀ Wasserstoff oder C₁₋₆-Alkyl ist; und R₁₃ ausgewählt ist aus Halogen, C₁₋₆-Alkyl, C₁₋₆-Alkoxy, Halogen-substituiertem C₁₋₆-Alkyl und Halogen-substituiertem C₁₋₆-Alkoxy.

4. Verbindung nach Anspruch 1, wobei R₁ ausgewählt ist aus: 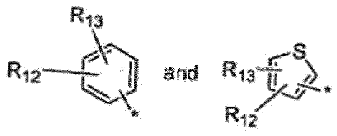 wobei R₁₂ ausgewählt ist aus Wasserstoff, Phenyl und Cyclohexyl; wobei jedes Phenyl oder Cyclohexyl von R₁₂ optional mit Methyl substituiert ist; und R₁₃ ausgewählt ist aus Trifluormethyl, Methyl und Ethyl.

5. Verbindung nach Anspruch 4, ausgewählt aus 3-{6-[3-(2-trifluormethylbiphenyl-4-yl)-[1,2,4]oxadiazol-5-yl]-3,4-dihydro-1H-isochinolin-2-yl}propionsäure, 3-[6-(2-Trifluormethylbiphenyl-4-yloxymethyl)-3,4-dihydro-1H-isochinolin-2-yl]propionsäure, 3-{5-[5-(2-Trifluoromethylbiphenyl-4-yl)isoxazol-3-yl]-1,3-dihydroisoindol-2-yl}propionsäure, 3-{5-[5-(2-Trifluormethylbiphenyl-4-yl)-[1,2,4]oxadiazol-3-yl]-1,3-dihydro-isoindol-2-yl}propionsäure, 3-{5-[5-(2-Trifluormethylbiphenyl-4-yl)-[1,3,4]oxadiazol-2-yl]-1,3-dihydroisoindol-2-yl}propionsäure, 3-[2-(2-Trifluormethylbiphenyl-4-yl)-5,7-dihydrooxazolo[4,5-f]isoindol-6-yl]propionsäure, 3-{7-[5-(2-Trifluormethylbiphenyl-4-yl)-[1,2,4]oxadiazol-3-yl]-3,4-dihydro-1H-isochinolin-2-yl}propionsäure, 3-{6-[5-(2-Trifluormethylbiphenyl-4-yl)-[1,2,4]oxadiazol-3-yl]-3,4-dihydro-1H-isochinolin-2-yl}propionsäure, 3-{6-[5-(2-Trifluormethylbiphenyl-4-yl)-[1,3,4]oxadiazol-2-yl]-3,4-dihydro-1H-isochinolin-2-yl}propionsäure, 3-[6-(3-Trifluormethylbenzyloxy)-3,4-dihydro-1H-isochinolin-2-yl]propionsäure, 3-[6-(4-Cyclohexyl-3-trifluormethylbenzyloxy)-3,4-dihydro-1H-isochinolin-2-yl]propionsäure, 3-(6-(2-Trifluormethylbiphenyl-4-ylmethoxy)-3,4-dihydro-1H-isochinolin-2-yl]propionsäure, 3-[7-(2-Trifluormethylbiphenyl-4-ylmethoxy)-3,4-dihydro-1H-isochinolin-2-yl]propionsäure, 3-[7-(4-Cyclohexyl-3-trifluormethylbenzyloxy)-3,4-dihydro-1H-isochinolin-2-yl]propionsäure, 3-[6-(4-Cyclohexyl-3-methylphenoxymethyl)-3,4-dihydro-1H-isochinolin-2-yl]propionsäure, 3-[6-(4-Cyclohexyl-3-ethylphenoxymethyl)-3,4-dihydro-1H-isochinolin-2-yl]propionsäure, 3-[6-(2-Ethylbiphenyl-4-yloxymethyl)-3,4-dihydro-1H-isochinolin-2-yl]propionsäure und 3-[6-(2-Ethyl-3'-methylbiphenyl-4-yloxymethyl)-3,4-dihydro-1H-isochinolin-2-yl]propionsäure.

6. Pharmazeutische Zusammensetzung, die eine therapeutisch wirksame Menge einer Verbindung nach Anspruch 1 in Kombination mit einem pharmazeutisch annehmbaren Exzipienten umfasst.

7. Pharmazeutische Zusammensetzung nach Anspruch 6, die eine therapeutisch wirksame Menge einer Verbindung nach einem der Ansprüche 1-5 in Kombination mit einem immunsuppressiven Mittel, einem immunmodulierenden Mittel oder anderem entzündungshemmenden Mittel umfasst.

8. Pharmazeutische Zusammensetzung nach Anspruch 6, die eine therapeutisch wirksame Menge einer Verbindung nach einem der Ansprüche 1-5 in Kombination mit einem oder mehreren der Folgenden umfasst:
i. einem Aromatasehemmer,
ii. einem Anti-Östrogen, einem Anti-Androgen (insbesondere im Fall von Prostatakrebs) oder einem Gonadorelin-Agonist,
iii. einem Topoisomerase-I-Hemmer oder einem Topoisomerase-II-Hemmer,
iv. einem Mikrotubuli-aktiven Mittel, einem Alkylierungsmittel, einem antineoplastischen Antimetabolit oder einer Platinverbindung,
v. einer Verbindung, die auf eine Protein- oder Lipidkinaseaktivität oder eine Protein- oder Lipidphosphataseaktivität gerichtet ist oder eine solche verringert, einer weiteren antiangiogenen Verbindung oder einer Verbindung, die Zelldifferenzierungsprozesse induziert,
vi. einem Bradykinin-1-Rezeptor oder einem Angiotensin-II-Antagonist,
vii. einem Cyclooxygenase-Hemmer, einem Bisphosphonat, einem Histon-Deacetylase-Hemmer, einem Heparanase-Hemmer (verhindert den Heparansulfatabbau), z.B. PI-88, einem Modifikator der biologischen Antwort, vorzugsweise einem Lymphokin oder Interferonen, einem Ubiquitinierungs-Hemmer oder einem Hemmer, der antiapoptotische Wege blockt,
viii. einem Hemmer von onkogenen Ras-Isoformen, z.B. H-Ras, K-Ras oder N-Ras, oder einem Farnesyl-Transferase-Hemmer, z.B. L-744,832 oder DK8G557,
ix. einem Telomerase-Hemmer, z.B. Telomestatin,
x. einem Protease-Hemmer, einem Matrix-Metalloproteinase-Hemmer, einem Methionin-Aminopeptidase-Hemmer, z.B. Bengamid oder einem Derivat davon oder einem Proteosom-Hemmer, z.B. PS-341, und/oder
xi. einem mTOR-Hemmer.

9. Verbindung nach einem der Ansprüche 1 bis 5 oder eine Zusammensetzung nach den Ansprüchen 6-8 zur Verwendung bei der Verhinderung oder Behandlung von Störungen oder Erkrankungen, die durch Lymphozyten vermittelt werden, zur Verhinderung oder Behandlung einer akuten oder chronischen Transplantatabstoßung oder T-Zell-vermittelten Entzündungs- oder Autoimmunerkrankung, zur Hemmung oder Kontrolle deregulierter Angiogenese oder zur Verhinderung oder Behandlung von Erkrankungen, die durch einen Neo-Angiogenese-Prozess vermittelt werden oder mit deregulierter Angiogenese in einem Subjekt assoziiert sind, umfassend Verabreichen an das Subjekt, das derer bedarf, einer wirksamen Menge einer Verbindung nach Anspruch 1 oder eines pharmazeutisch annehmbaren Salzes davon.

10. Verbindung oder Zusammensetzung nach Anspruch 9, wobei die Krankheit oder Störung ausgewählt ist aus Transplantationsabstoßung, verzögerter Transplantatfunktion, Graft-versus-Host-Krankheit, rheumatoider Arthritis, systemischem Lupus erythematodes, Hashimoto-Thyreoiditis, Multipler Sklerose, Myasthenia gravis, Diabetes Typ I oder II und der damit verbundenen Störungen, Vaskulitis, perniziöser Anämie, Sjögrensyndrom, Uveitis, Psoriasis, Graves Ophthalmopathie, Alopecia areata und anderen, allergischen Erkrankungen, entzündlicher Darmerkrankung, Morbus Crohn, Colitis ulcerosa, intrinsischem Asthma, entzündlicher Lungenschädigung, entzündlicher Leberschädigung, entzündlicher glomerulärer Verletzung, Arteriosklerose, Osteoarthrose, gereizter Kontaktdermatitis, ekzematösen Dermatitiden, seborrhoischer Dermatitis, Hautmanifestationen von immunologisch vermittelten Erkrankungen, entzündlicher Augenerkrankung, Keratokonjunktivitis, Myokarditis, Hepatitis, Ischämie/Reperfusionsverletzung, Schlaganfall, Darm-Ischämie, Nierenversagen, hämorrhagischem Schock, traumatischem Schock, T-Zell-Lymphomen, T-Zell-Leukämien, Infektionskrankheiten und Altersdemenz.

## Revendications

1. Composé de formule I : 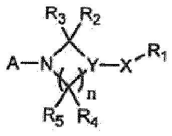 dans lequel
n est 1 ou 2 ;
A est -(CH₂)₂C(O)OH ;
X est une liaison ou est choisi parmi [1,2,4]oxadiazole, -CH₂O-, -OCH₂-, des isoxazoles et [1,3,4]oxadiazole ;
Y est choisi parmi un groupe phényle et benzooxazolyle ;
R₁ est choisi parmi un groupe aryle en C₆₋₁₀ et hétéroaryle en C₂₋₉, dans lequel un groupe aryle ou hétéroaryle quelconque de R₁ est éventuellement substitué par un radical choisi parmi un groupe aryle en C₆₋₁₀-alkyle en C₀₋₄, hétéroaryle en C₂₋₉-alkyle en C₀₋₄, cycloalkyle en C₃₋₈-alkyle en C₀₋₄, hétérocycloalkyle en C₃₋₈-alkyle en C₀₋₄ ou alkyle en C₁₋₆; dans lequel un groupe aryle, hétéroaryle, cycloalkyle ou hétérocycloalkyle quelconque de R₁ peut éventuellement être substitué par un à cinq radicaux choisis parmi un groupe halogéno, alkyle en C₁₋₆, alcoxy en C₁₋₆, alkyle en C₁₋₆ substitué par un groupe halogéno et alcoxy en C₁₋₆ substitué par un groupe halogéno ; et un groupe alkyle quelconque de R₁ peut éventuellement avoir un groupe méthylène remplacé par un atome ou un groupe choisi parmi -S-, -S(O)-, -S(O)₂-, -NR₇- et -O- ; dans lequel R₇ est choisi parmi l'hydrogène ou un groupe alkyle en C₁₋₆,
R₂, R₃, R₄ et R₅ sont l'hydrogène ; et sels, hydrates, solvates, dérivés N-oxyde, isomères optiques ou géométriques pharmaceutiquement acceptables choisis parmi des composés *cis,* des composés *trans* et des mélanges de ceux-ci.

2. Composé selon la revendication 1 dans lequel R₁ est un groupe phényle, naphtyle, furanyle ou thiényle éventuellement substitué par un groupe aryle en C₆₋₁₀-alkyle en C₀₋₄, hétéroaryle en C₂₋₉-alkyle en C₀₋₄, cycloalkyle en C₃₋₈-alkyle en C₀₋₄, hétérocycloalkyle en C₃₋₈-alkyle en C₀₋₄ ou alkyle en C₁₋₆; dans lequel un groupe aryle, hétéroaryle, cycloalkyle ou hétérocycloalkyle quelconque de R₁ peut éventuellement être substitué par un à cinq radicaux choisis parmi un groupe halogéno, alkyle en C₁₋₆, alcoxy en C₁₋₆, alkyle en C₁₋₆ substitué par un groupe halogéno et alcoxy en C₁₋₆ substitué par un groupe halogéno ; et un groupe alkyle quelconque de R₁ peut éventuellement avoir un groupe méthylène remplacé par un atome ou un groupe choisi parmi -S-, -S(O)-, -S(O)₂-, -NR₇- et -O- ; dans lequel R₇ est l'hydrogène ou un groupe alkyle en C₁₋₆.

3. Composé selon la revendication 1, dans lequel R₁ est choisi parmi : 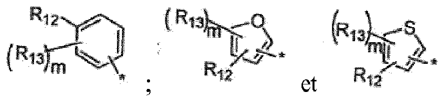 dans lequel l'astérisque est le point de liaison de R₁ avec X ; m est choisi parmi 1 et 2 ; R₁₂ est choisi parmi l'hydrogène, un groupe aryle en C₆₋₁₀-alkyle en C₀₋₄, hétéroaryle en C₂₋₉-alkyle en C₀₋₄, cycloalkyle en C₃₋₈-alkyle en C₀₋₄, hétérocycloalkyle en C₃₋₈-alkyle en C₀₋₄ ou alkyle en C₁₋₆ ; dans lequel un groupe aryle, hétéroaryle, cycloalkyle ou hétérocycloalkyle quelconque de R₁₂ peut éventuellement être substitué par un à trois radicaux choisis parmi un groupe halogéno, alkyle en C₁₋₆, alcoxy en C₁₋₆, alkyle en C₁₋₆ substitué par un groupe halogéno et alcoxy en C₁₋₆ substitué par un groupe halogéno ; et un groupe alkyle quelconque de R₁₂ peut éventuellement avoir un groupe méthylène remplacé par un atome ou un groupe choisi parmi -S-, -S(O)-, -S(O)₂-, -NR₁₀- et -O- ; dans lequel R₁₀ est l'hydrogène ou un groupe alkyle en C₁₋₆ ; et R₁₃ est choisi parmi un groupe halogéno, alkyle en C₁₋₆, alcoxy en C₁₋₆, alkyle en C₁₋₆ substitué par un groupe halogéno et alcoxy en C₁₋₆ substitué par un groupe halogéno.

4. Composé selon la revendication 1, dans lequel R₁ est choisi parmi : 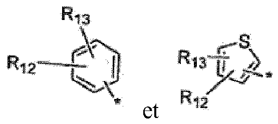 dans lequel R₁₂ est choisi parmi l'hydrogène, un groupe phényle et cyclohexyle ; dans lequel un groupe phényle ou cyclohexyle quelconque de R₁₂ est éventuellement substitué par un groupe méthyle ; et R₁₃ est choisi parmi un groupe trifluorométhyle, méthyle et éthyle.

5. Composé selon la revendication 4 choisi parmi l'acide 3-{6-[3-(2-trifluorométhyl-biphényl-4-yl)-[1,2,4]oxadiazol-5-yl]-3,4-dihydro-1H-isoquinoléin-2-yl{-propionique, l'acide 3-[6-(2-trifluorométhyl-biphényl-4-yloxyméthyl)-3,4-dihydro-1H-isoquinoléin-2-yl]-propionique, l'acide 3-{5-[5-(2-trifluorométhyl-biphényl-4-yl)-isoxazol-3-yl]-1,3-dihydro-isoindol-2-yl{-propionique, l'acide 3-{5-[5-(2-trifluorométhyl-biphényl-4-yl)-[1,2,4]oxadiazol-3-yl]-1,3-dihydro-isoindol-2-yl{-propionique, l'acide 3-{5-[5-(2-trifluorométhyl-biphényl-4-yl)-[1,3,4]oxadiazol-2-yl]-1,3-dihydro-isoindol-2-yl{-propionique, l'acide 3-[2-(2-trifluorométhyl-biphényl-4-yl)-5,7-dihydro-oxazolo[4,5-f]isoindol-6-yl]-propionique, l'acide 3-{7-[5-(2-trifluorométhyl-biphényl-4-yl)-[1,2,4]oxadiazol-3-yl]-3,4-dihydro-1H-isoquinoléin-2-yl}-propionique, l'acide 3-{6-[5-(2-trifluorométhyl-biphényl-4-yl)-[1,2,4]oxadiazol-3-yl]-3,4-dihydro-1H-isoquinoléin-2-yl}-propionique, l'acide 3-{6-[5-(2-trifluorométhyl-biphényl-4-yl)-[1,3,4]oxadiazol-2-yl]-3,4-dihydro-1H-isoquinoléin-2-yl{-propionique, l'acide 3-[6-(3-trifluorométhyl-benzyloxy)-3,4-dihydro-1H-isoquinoléin-2-yl]-propionique, l'acide 3-[6-(4-cyclohexyl-3-trifluorométhyl-benzyloxy)-3,4-dihydro-1H-isoquinoléin-2-yl]-propionique, l'acide 3-[6-(2-trifluorométhyl-biphényl-4-ylméthoxy)-3,4-dihydro-1H-isoquinoléin-2-yl]-propionique, l'acide 3-[7-(2-trifluorométhyl-biphényl-4-ylméthoxy)-3,4-dihydro-1H-isoquinoléin-2-yl]-propionique, l'acide 3-[7-(4-cyclohexyl-3-trifluorométhyl-benzyloxy)-3,4-dihydro-1H-isoquinoléin-2-yl]-propionique, l'acide 3-[6-(4-cyclohexyl-3-méthyl-phénoxyméthyl)-3,4-dihydro-1H-isoquinoléin-2-yl]-propionique, l'acide 3-[6-(4-cyclohexyl-3-éthyl-phénoxyméthyl)-3,4-dihydro-1H-isoquinoléin-2-yl]-propionique, l'acide 3-[6-(2-éthyl-biphényl-4-yloxyméthyl)-3,4-dihydro-1H-isoquinoléin-2-yl]-propionique et l'acide 3-[6-(2-éthyl-3'-méthyl-biphényl-4-yloxyméthyl)-3,4-dihydro-1H-isoquinoléin-2-yl]-propionique.

6. Composition pharmaceutique comprenant une quantité thérapeutiquement efficace d'un composé selon la revendication 1 en combinaison avec un excipient pharmaceutiquement acceptable.

7. Composition pharmaceutique selon la revendication 6, comprenant une quantité thérapeutiquement efficace d'un composé selon l'une quelconque des revendications 1 à 5, en combinaison avec un agent immunosuppresseur, un agent immunomodulateur ou un autre agent anti-inflammatoire.

8. Composition pharmaceutique selon la revendication 6, comprenant une quantité thérapeutiquement efficace d'un composé selon l'une quelconque des revendications 1 à 5, en combinaison avec un ou plusieurs parmi :
i. un inhibiteur de l'aromatase,
ii. un anti-oestrogène, un anti-androgène (notamment dans le cas du cancer de la prostate) ou un agoniste de gonadolibérine,
iii. un inhibiteur de la topoisomérase I ou un inhibiteur de la topoisomérase II,
iv. un agent actif à microtubules, un agent alkylant, un antimétabolite antinéoplasique ou un composé de platine,
v. un composé ciblant/réduisant une activité protéine ou lipide kinase ou une activité protéine ou lipide phosphatase, un autre composé anti-angiogénique ou un composé qui induit les processus de différenciation cellulaire,
vi. un récepteur de la bradykinine 1 ou un antagoniste de l'angiotensine II,
vii. un inhibiteur de la cyclooxygénase, un bisphosphonate, un inhibiteur de l'histone-désacétylase, un inhibiteur de l'héparanase (prévient la dégradation d'héparan sulfate), par exemple PI-88, un modificateur de la réponse biologique, de préférence une lymphokine ou des interférons, un inhibiteur d'ubiquitination, ou un inhibiteur qui bloque les voies de signalisation anti-apoptotiques,
viii. un inhibiteur des isoformes oncogènes de Ras, par exemple H-Ras, K-Ras ou N-Ras, ou un inhibiteur de la farnésyl-transférase, par exemple L-744 832 ou DK8G557,
ix. un inhibiteur de la télomérase, par exemple la téloméstatine,
x. un inhibiteur de protéases, un inhibiteur de la métalloprotéinase matricielle, un inhibiteur de la méthionine aminopeptidase, par exemple un bengamide ou un dérivé de celui-ci, ou un inhibiteur du protéosome, par exemple PS-341, et/ou
xi. un inhibiteur de mTOR.

9. Composé selon l'une quelconque des revendications 1 à 5, ou composition selon l'une quelconque des revendications 6 à 8, pour une utilisation dans la prévention ou le traitement de troubles ou maladies médiés par les lymphocytes, dans la prévention ou le traitement du rejet de greffe aigu ou chronique ou de maladies inflammatoires médiées par les lymphocytes T ou auto-immunes, dans l'inhibition ou le contrôle de l'angiogenèse dérégulée, ou dans la prévention ou le traitement de maladies médiées par un processus de néo-angiogenèse ou associées à l'angiogenèse dérégulée chez un sujet, comprenant l'administration au sujet nécessitant celui-ci d'une quantité efficace d'un composé selon la revendication 1 ou d'un sel pharmaceutiquement acceptable de celui-ci.

10. Composé ou composition selon la revendication 9, dans lequel/laquelle la maladie ou le trouble est choisi(e) parmi le rejet de greffe, le retard de reprise de fonction du greffon, la maladie du greffon contre l'hôte, la polyarthrite rhumatoïde, le lupus érythémateux disséminé, la thyroïdite chronique de Hashimoto, la sclérose en plaques, la myasthénie gravis, le diabète de type I ou II et les troubles qui lui sont associés, la vascularite, l'anémie pernicieuse, le syndrome de Sjoegren, l'uvéite, le psoriasis, l'ophtalmopathie de Graves, la pelade et d'autres, les maladies allergiques, l'affection abdominale inflammatoire, la maladie de Crohn, la rectocolite hémorragique, l'asthme intrinsèque, les lésions pulmonaires inflammatoires, les lésions hépatiques inflammatoires, les lésions glomérulaires inflammatoires, l'athérosclérose, l'arthrose, la dermatite irritative de contact, les dermites eczémateuses, la dermatite séborrhéique, les manifestations cutanées des troubles à médiation immunologique, les pathologies oculaires inflammatoires, la kérato-conjonctivite, la myocardite, l'hépatite, les lésions d'ischémie reperfusion, l'accident vasculaire cérébral, l'ischémie intestinale, l'insuffisance rénale, le choc hémorragique, le choc traumatique, les lymphomes à lymphocytes T, les leucémies à lymphocytes T, les maladies infectieuses et la démence sénile.
